# EUROPEAN PATENT SPECIFICATION

(11) **EP 3 784 240 B1**
(45) Date of publication and mention of the grant of the patent: **20.09.2023**
(21) Application number: 19737873.0
(22) Date of filing: 24.04.2019
(51) Int. Cl.: A61K 38/17, A61K 31/497, A61K 31/192, A61K 31/4709, A61K 31/609, A61K 39/395, A61P 35/00, G01N 33/574

(54) **WNT6 AS GLIOBLASTOMA ONCOGENIC BIOMARKER, AND USES OF INHIBITORS THEREOF FOR TREATING WNT6-OVEREXPRESSING GLIOBLASTOMA**
WNT6 ALS GLIOBASTOM TUMORMARKER, UND VERWENDUNG VON INHIBITOREN DAVON ZUR BEHANDLUNG VON WNT6-ÜBEREXPRIMIERENDES GLIOBLASTOM
WNT6 COMME BIOMARQUEUR ONCOGÈNIQUE DU GLIOBLASTOME, ET UTILISATIONS D'INHIBITEURS ASSOCIÉS POUR TRAITER LE GLIOBLASTOME SUREXPRIMANT WNT6

(30) Priority: 24.04.2018 PT 2018110707
(43) Date of publication of application: 03.03.2021
(73) Proprietor: Universidade do Minho, 4704-553 Braga (PT)
(72) Inventor: MARQUES DA COSTA, Bruno Filipe, 4715-352 Braga (PT); SARAIVA GONÇALVES, Céline, 4730-390 Pico De Regalados (PT); CARVALHO DE SOUSA, Nuno Jorge, 4200-139 Porto (PT)
(74) Representative: Patentree
(86) International application number: PCT/IB2019/053396
(87) International publication number: WO 2019/207512

(56) References cited:
- US-A1- 2009 099 062
- US-A1- 2015 174 086
- US-A1- 2016 129 030
- US-A1- 2017 247 465
- US-A1- 2017 368 158
- US-B1- 8 853 274
- US-B2- 9 457 081
- J. LIU ET AL: "Targeting Wnt-driven cancer through the inhibition of Porcupine by LGK974", PROCEEDINGS OF THE NATIONAL ACADEMY OF SCIENCES, vol. 110, no. 50, 25 November 2013 (2013-11-25), pages 20224-20229, XP055121007, ISSN: 0027-8424, DOI: 10.1073/pnas.1314239110
- TABATABAI ROYA ET AL: "Targeting the Wnt Pathway in Cancer: A Review of Novel Therapeutics", TARGETED ONCOLOGY, SPRINGER PARIS, PARIS, vol. 12, no. 5, 26 June 2017 (2017-06-26), pages 623-641, XP036327562, ISSN: 1776-2596, DOI: 10.1007/S11523-017-0507-4 [retrieved on 2017-06-26]
- GONÇALVES C ET AL: "Regulation of WNT6 by HOXA9 in glioblastoma: functional and clinical relevance", EUROPEAN JOURNAL OF CANCER, vol. 61, no. S1, 248, 2 July 2016 (2016-07-02), - 12 July 2016 (2016-07-12), pages S45-S46, XP029633989, EACR24 ISSN: 0959-8049, DOI: 10.1016/S0959-8049(16)61151-6
- ABIGAIL K. SUWALA ET AL: "Clipping the Wings of Glioblastoma: Modulation of WNT as a Novel Therapeutic Strategy", JOURNAL OF NEUROPATHOLOGY AND EXPERIMENTAL NEUROLOGY., vol. 75, no. 5, 15 March 2016 (2016-03-15) , pages 388-396, XP055627850, NEW YORK, NY. ISSN: 0022-3069, DOI: 10.1093/jnen/nlw013
- ISHAQ N. KHAN ET AL: "Current and emerging biomarkers in tumors of the central nervous system: Possible diagnostic, prognostic and therapeutic applications", SEMINARS IN CANCER BIOLOGY., vol. 52, 31 July 2017 (2017-07-31), pages 85-102, XP055629877, US ISSN: 1044-579X, DOI: 10.1016/j.semcancer.2017.07.004

## Description

### Technical field

The present disclosure relates to a novel biomarker for use in human glioblastoma, namely to a compound inhibiting WNT6 biomarker for the treatment of glioblastoma with WNT6 over-expression, preferably proneural glioblastoma with WNT6 over-expression, classic glioblastoma with WNT6 over-expression, neural glioblastoma with WNT6 over-expression, or mesenchymal glioblastoma with WNT6 over-expression,

The present disclosure also relates to a WNT pathway inhibitor for use in a method of treating glioblastoma with WNT6 over-expression, preferably proneural glioblastoma with WNT6 over-expression, classic glioblastoma with WNT6 over-expression, neural glioblastoma with WNT6 over-expression, or mesenchymal glioblastoma with WNT6 over-expression.

### Background Art

Glioblastoma (GBM) is the most lethal tumor of the central nervous system in adults. Despite all the progresses in the understanding of the molecular tumorigenic mechanisms and the improvements in neuroimaging technologies, surgery and adjuvant treatments, patients with GBM still exhibit a rapid progression and present a median survival of approximately 15 months after diagnosis, a scenario that has not changed significantly in the last decades. In combination with their resistance to most conventional therapies, GBMs are highly infiltrative and diffuse, making a complete surgical resection virtually impossible. In this context, understanding the key underlying molecular mechanisms contributing to the aggressiveness of this tumor may be a critical step in the design of new and more effective precision therapies.

WNT proteins are pleiotropic in their activity, with roles from neurogenesis to stem cell proliferation. Unsurprisingly, WNT aberrant activity has been implicated in various human cancers, including in glioma, and is particularly relevant in the context of cancer stem cells (CSC), which have been pointed as critical for cancer recurrence and resistance to radiochemotherapy. Therefore, novel therapeutic strategies targeting particular components of the WNT pathway have been widely explored. The WNT6 ligand is an activator of the WNT pathway, which only recently was associated with chemoresistance in gastric and bladder cancers (1, 2), with poor prognosis of esophageal squamous cell carcinoma patients (3), and with increased risk to colorectal adenoma (4). Importantly, no studies to date have explored the relevance of WNT6 in human glioma.

### General Description

Glioblastoma (GBM) is a dramatic brain cancer that affects all ages, from children to adults, remaining universally fatal. Understanding the underlying oncogenic molecular events in GBM is thus a crucial step towards better therapies. The WNT pathway is aberrantly activated in several human cancers, including GBM, and is associated with tumor aggressiveness. While the relevance of a variety of WNT signaling molecules has been established, nothing is known about the role of WNT6 in GBM.

The present disclosure provides novel molecular, functional, mechanistic and clinical data on the relevance of WNT6 in GBM. Herein it is shown that WNT6 is significantly overexpressed in GBMs, as compared to lower-grade gliomas and non-tumor brain, at the mRNA and protein levels. Functionally, WNT6 increases typical oncogenic activities in GBM cells, including viability, proliferation, stem cell capacity, invasion, migration, and resistance to temozolomide chemotherapy. In an *in vivo* orthotopic GBM model, WNT6 leads to shorter overall survival of mice, and was associated with increased tumor cell proliferation, stem cell capacity, and anti-apoptotic features of the tumors. Mechanistically, WNT6 contributes to activate typical oncogenic signaling pathways, including RTK and STAT, which intertwined with the WNT pathway may be critical effectors of WNT6-associated aggressiveness in GBM.

Furthermore, the present disclosure provides the first evidence of the clinical prognostic value of WNT6 in GBM patients from several cohorts, using a variety of detection techniques, consistently implicating high levels of WNT6 as a novel independent prognostic biomarker of shorter survival. Together, the findings now disclosed establish WNT6 as a novel oncogene in GBM, and new opportunities to develop novel therapeutic approaches to treat this highly-aggressive tumor are, therefore, also disclosed.

In this disclosure, it is shown that WNT6 is overexpressed in GBM (grade IV glioma), and displays typical hallmark oncogenic functions in both *in vitro* and *in vivo* GBM models by affecting the activity of classic oncogenic signalling pathways, including WNT, RTK and STAT pathways. Critically, it is also provided data from 6 independent GBM patient cohorts establishing WNT6 as a prognostic biomarker associated with shorter overall survival (OS).

The present disclosure relates to a biomarker of the WNT pathway for use in the treatment of cancer, preferably wherein the biomarker is WNT6.

Another aspect of the present disclosure also relates to a WNT pathway inhibitor for use in a method of treating glioblastoma with WNT6 over-expression, preferably proneural glioblastoma with WNT6 over-expression, classic glioblastoma with WNT6 over-expression, neural glioblastoma with WNT6 over-expression, or mesenchymal glioblastoma with WNT6 over-expression, in a subject in need thereof, wherein the method comprises:
- determining the levels of the WNT6 biomarker in a sample from the subject, and
- comparing the level of the WNT6 biomarker in the sample to a predetermined level of WNT6; wherein if the level of the WNT6 in the sample is higher than the predetermined level of the WNT6, then the subject is administered a therapeutically effective amount of a WNT6inhibitor.

The compound inhibitor of WNT6 is selected from the following list:
vantictumab (OMP-18RS; CAS Number: 1345009-45-1), GNF-1331 (CAS Number: 603101-22-0) , ipafricept (OMP-54F28; CAS Number: 1391727-24-4), XAV-939 (CAS Number: 284028-89-3), IWR-1 (CAS Number: 1127442-82-3), pyrvinium (CAS Number: 7187-62-4), DKN-01 (LY2812176), IWP-2 (CAS Number: 686770-61-6), WNT974 (LGK974; CAS Number: 1243244-14-5), niclosamide (CAS Number: 50-65-7), sulindac (CAS Number: 38194-50-2), NSC668036 (CAS Number: 144678-63-7), J01-017a, PRI-724 (CAS Number: 1198780-38-9), ICG-001 (CAS Number: 847591 - 62 - 2), PKF115-584 (Calphostin C; CAS Number: 121263-19-2), PKF118-310 (Toxoflavin, Xanthothricin; CAS Number: 84-82-2), NCB-0846 (CAS Number: 1792999-26-8), CGP049090 (Cercosporin; CAS Number: 35082-49-6), StAX-35, NVP-TNKS656, JW74, Foxy-5 (CAS Number: 881188-51-8), OTSA 101 (OncoTherapy Science), CWP232291 (JW Pharmaceutical), ETC 159 (CAS Number: 1638250-96-0), OMP131R10 (Rosmantuzumab; CAS Number: 1684393-04-1), RO4929097 (CAS Number: 847925-91-1), MK0752 (CAS Number: 471905-41-6), PF-03084014 (CAS Number: 1290543-63-3), or combinations thereof. Preferably for better results, the compound inhibitor of WNT6 is WNT974 (LGK974; CAS Number: 1243244-14-5).

Another aspect of the present disclosure also relates to a pharmaceutical composition for use in the treatment of glioblastoma with WNT6 over-expression comprising the compound described in the present disclosure and a pharmaceutical acceptable excipient.

Another aspect of the present disclosure also relates to a kit for use in the treatment of glioblastoma with WNT6 over-expression, preferably proneural glioblastoma with WNT6 over-expression, classic glioblastoma with WNT6 over-expression, neural glioblastoma with WNT6 over-expression, or mesenchymal glioblastoma with WNT6 over-expression, comprising the pharmaceutical composition or a compound described in the present disclosure.

The disclosure also relates to a compound selected from the following list:
vantictumab (OMP-18RS; CAS Number: 1345009-45-1), GNF-1331 (CAS Number: 603101-22-0) , ipafricept (OMP-54F28; CAS Number: 1391727-24-4), XAV-939 (CAS Number: 284028-89-3), IWR-1 (CAS Number: 1127442-82-3), pyrvinium (CAS Number: 7187-62-4), DKN-01 (LY2812176), IWP-2 (CAS Number: 686770-61-6), WNT974 (LGK974; CAS Number: 1243244-14-5), niclosamide (CAS Number: 50-65-7), sulindac (CAS Number: 38194-50-2), NSC668036 (CAS Number: 144678-63-7), J01-017a, PRI-724 (CAS Number: 1198780-38-9), ICG-001 (CAS Number: 847591 - 62 - 2), PKF115-584 (Calphostin C; CAS Number: 121263-19-2), PKF118-310 (Toxoflavin, Xanthothricin; CAS Number: 84-82-2), NCB-0846 (CAS Number: 1792999-26-8), CGP049090 (Cercosporin; CAS Number: 35082-49-6), StAX-35, NVP-TNKS656, JW74, Foxy-5 (CAS Number: 881188-51-8), OTSA 101 (OncoTherapy Science), CWP232291 (JW Pharmaceutical), ETC 159 (CAS Number: 1638250-96-0), OMP131R10 (Rosmantuzumab; CAS Number: 1684393-04-1), RO4929097 (CAS Number: 847925-91-1), MK0752 (CAS Number: 471905-41-6), PF-03084014 (CAS Number: 1290543-63-3), or combinations thereof; and wherein the compound is an inhibitor of WNT6, for use in the treatment of glioblastoma with WNT6 over-expression, preferably proneural glioblastoma with WNT6 over-expression, classic glioblastoma with WNT6 over-expression, neural glioblastoma with WNT6 over-expression, or mesenchymal glioblastoma with WNT6 over-expression.

The disclosure further relates to a kit comprising a biomarker of WNT pathway, wherein the biomarker is WNT6.

### Brief Description of the Drawings

The following figures provide preferred embodiments for illustrating the description and should not be seen as limiting the scope of disclosure.

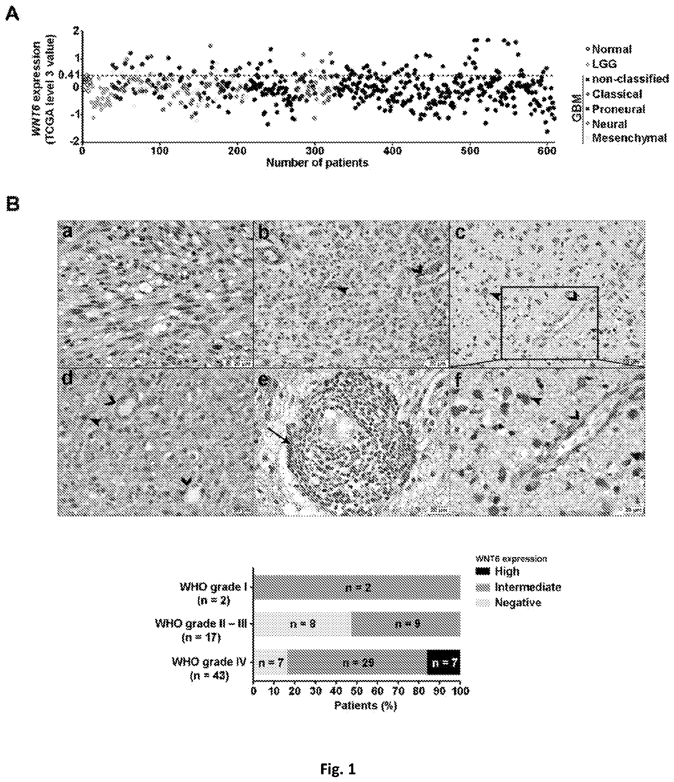

**Figure 1****.** WNT6 is overexpressed at the mRNA and protein levels in GBM. **(A)** Expression levels of WNT6 in 27 lower-grade gliomas (LGG; grey dots), 572 glioblastomas (GBM, WHO grade IV; black and colored dots) and 10 unmatched normal brains (black unfilled dots) from TCGA. GBM molecular subtypes are represented in colors (red = classical; blue = proneural; green = neural; yellow = mesenchymal). WNT6 is overexpressed (TCGA data level 3 values ≥ 0.41; above red dashed line) in 16% (n = 89) of GBM samples. **(B)** WNT6 protein expression in WHO grades I-IV glioma samples from Hospital Santo António cohort assessed by IHC (n = 63; p = 0.037; chi-squared test). Representative images are shown for a WNT6-negative GBM (a), high WNT6 expression in GBM (b) and WHO grade III anaplastic oligoastrocytoma (c), and a WHO grade II diffuse oligodendroglioma with intermediate levels of WNT6 expression (d). WNT6 staining was mostly cytoplasmic in glioma cells (closed arrowheads) and not present in lymphocytes (e; arrow), being almost exclusively negative for endothelial cells (b-d and f; open arrowheads). Bottom graph summarizes IHC data for the whole dataset.

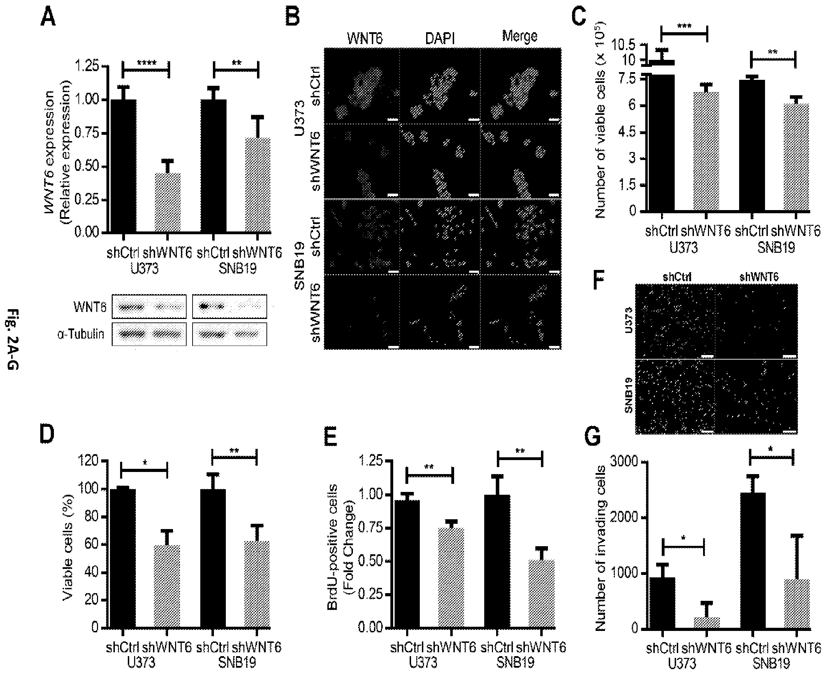

**Figure 2****.** WNT6 promotes GBM aggressiveness *in vitro.* (A and B). The efficiency of WNT6 silencing in U373 and SNB19 glioblastoma cells was analyzed by qRT-PCR and WB **(A),** and immunofluorescence **(B). (A)** Top: *WNT6* expression levels were normalized to *TBP.* Bottom: WB images are representative of 3 independent assays; α-tubulin was used as reference protein. **(B)** DAPI was used to stain the nucleus (40x magnification; scale bar = 50 µm). **(C and D)** Cell viability was measured by trypan blue **(C)** and MTT **(D)** assays in shCtrl and shWNT6 cells. **(E)** Cell proliferation was evaluated by BrdU incorporation. **(F and G)** Matrigel invasion assays were used to assess the cells' invasion capacity. Representative images (**F**; cell nuclei stained with DAPI; scale bar = 250 µm) and quantification **(G)** of invasive U373 and SNB19 shCtrl and shWNT6 cells. **(H** - **K)** Migration (wound healing) assays were performed in U373 **(H and I)** and SNB19 **(J and K)** shCtrl and shWNT6 cells to assess the cells' migration capacity. Representative micrographs throughout time for U373 **(H)** and SNB19 **(J)** cells (40x magnification; scale bar = 250 µm). The wound gap was measured and expressed as a percentage of wound closure for U373 **(I)** and SNB19 **(K)** cells (**** *p* <0.001; two-way ANOVA post-hoc Sidak's test). Results represent data from at least 3 independent experiments (mean ± SD). *, *p* < 0.05; **, *p* < 0.05; ***, *p* < 0.005 and ^{∗∗∗∗}, *p* < 0.001 (otherwise stated, a two-sided unpaired t-test with Welch's correction being applied when homoscedasticity was not verified).

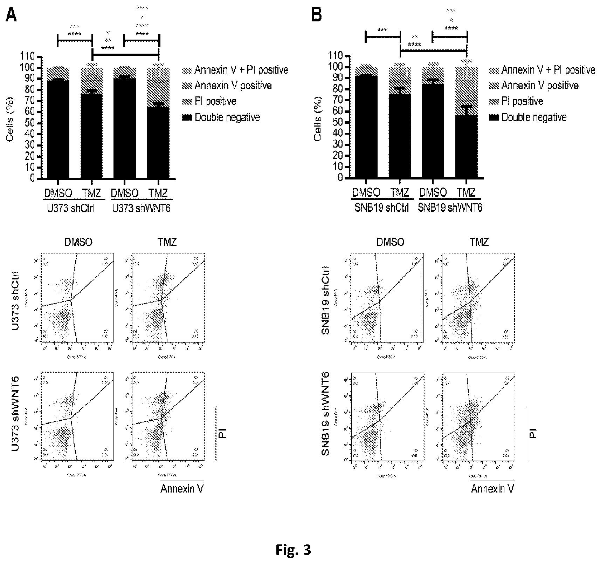

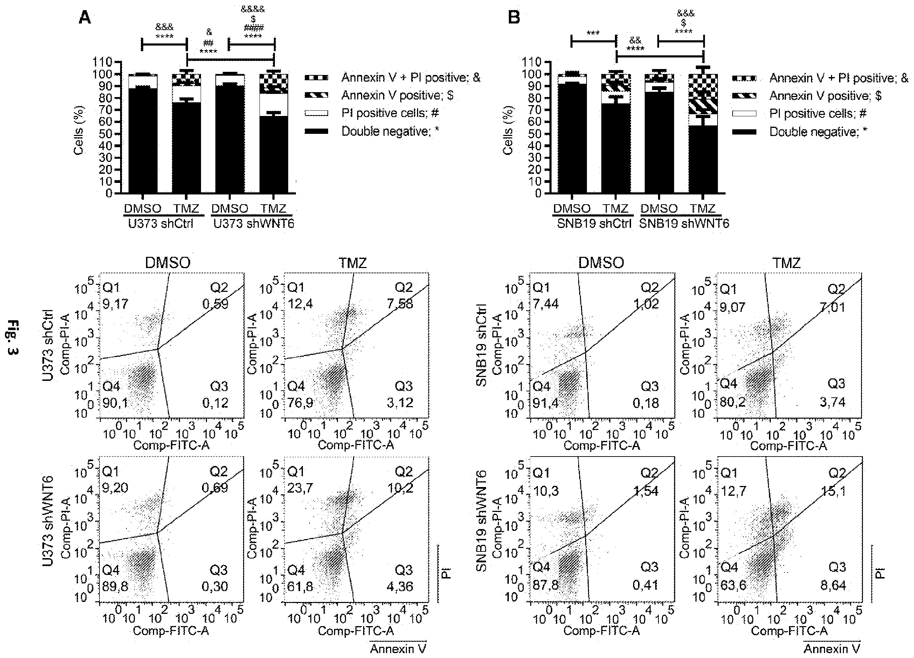

**Figure 3****.** WNT6 promotes resistance to temozolomide chemotherapy in GBM cells. **(A and B)** U373 **(A)** and SNB19 **(B)** shCtrl- and shWNT6-transfected cells were treated with vehicle or TMZ for 6 days, and cell death was measured by Annexin-V and PI. The graphs present data of 3 independent assays (mean ± SD). Representative dot plots are displayed below. *, *p* <0.05; **, *p* <0.05; ***, *p* < 0.001 and ****, *p* < 0.0001 (two-way ANOVA post-hoc Tukey's test).

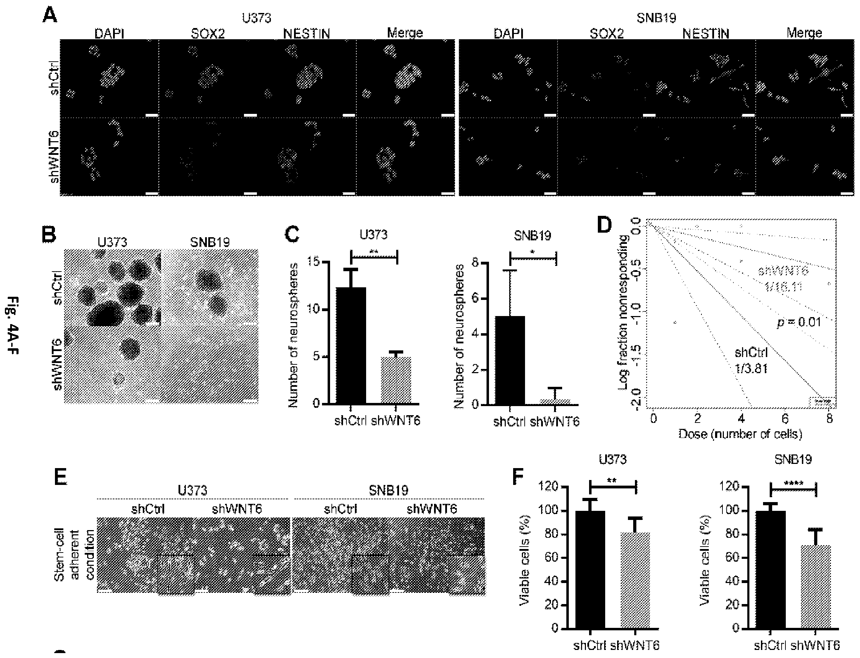

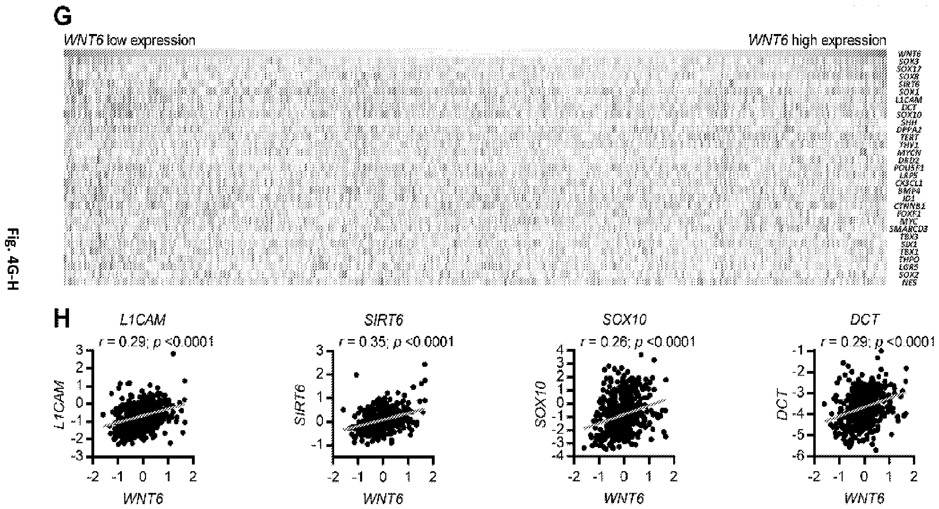

**Figure 4****.** WNT6 silencing decreases GBM stem cell features. **(A)** SOX2 and NESTIN double-immunostaining in U373 (left) and SNB19 (right) shCtrl/shWNT6 cells. DAPI was used to stain cell nucleus (40x magnification; scale bar = 50 µm). **(B)** Representative images of U373 (left) and SNB19 (right) shCtrl/shWNT6 neurospheres at days 10 and 15 after plating, respectively (40x magnification; scale bar = 250 µm). **(C)** Quantification of neurospheres formation (n = 3 independent assays; mean ± SD; *, *p* < 0.05; **, *p* < 0.01, two-sided unpaired *t*-test with Welch's correction being applied when homoscedasticity was not verified). **(D)** Limiting dilution assays in U373 shCtrl (black) and shWNT6 (red) cells to assess their sphere-forming capacity. The graph is representative of 3 independent assays with similar results. The trend lines represent the estimated active cell frequency (*p* = 0.01, likelihood ratio test). **(E)** Representative images of U373 (left) and SNB19 (right) shCtrl/shWNT6 cells cultured under stem cell adherent conditions (scale bar = 250 µm). **(F)** MTT assay evaluating U373 (left) and SNB19 (right) shCtrl/shWNT6 cell viability when cultured under adherent stem cell conditions (n = 3 independent assays; mean ± SD; **, *p* < 0.01; **** *p* < 0.0001; two-sided unpaired *t*-test with Welch's correction being applied when homoscedasticity was not verified). **(G)** Heatmap representation of expression levels of *WNT6* and 30 stem cell-related genes in 573 GBM patients' samples from TCGA. Each column corresponds to a patient and each line to a gene. Expression values increase from darker blue (low levels) to darker red (high levels). **(H)** Correlation graphs between *WNT6* expression (x-axis) and the expression of stem cell genes selected based on the heatmap (y-axis; Pearson's correlation test *r* and *p* values are indicated).

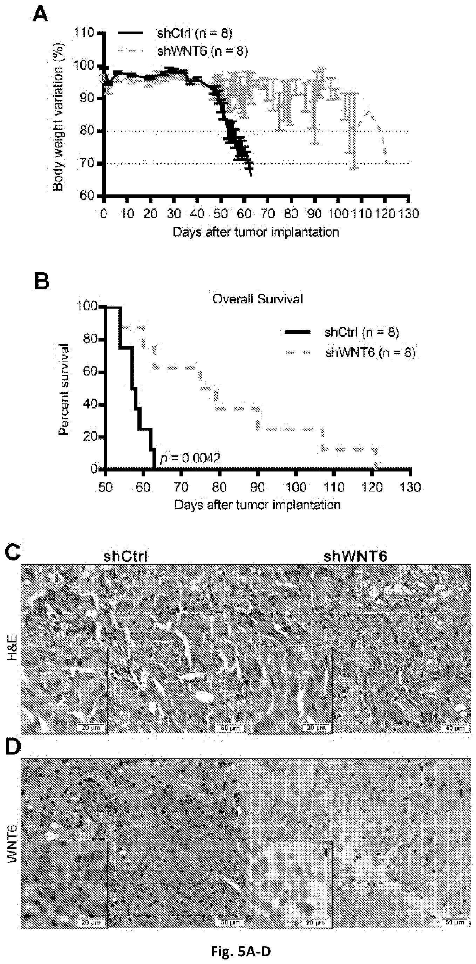

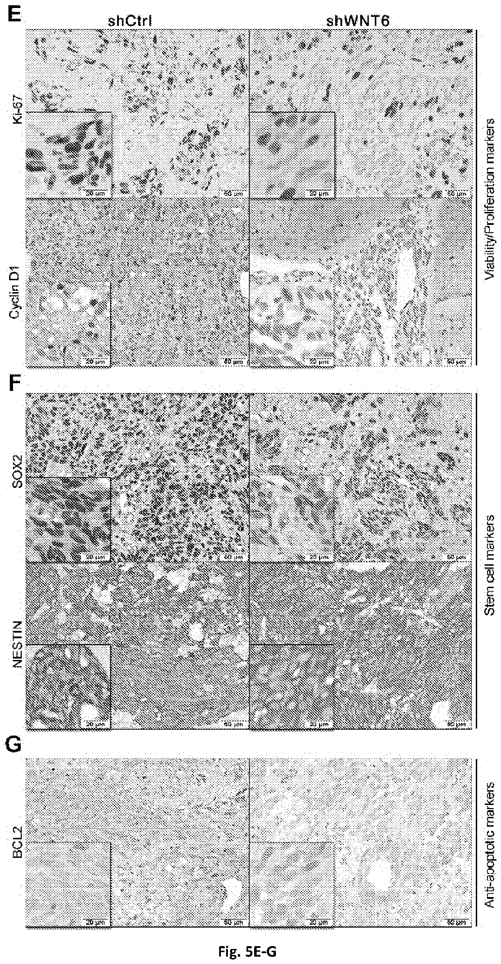

**Figure 5****.** WNT6 accelerates GBM-associated death in an intracranial mice model. U373 transfected cells were orthotopically injected in the brain of NSG mice (n = 8 per group). **(A)** Weight curves after tumor implantation. **(B)** Kaplan-Meier overall survival curves of mice (*p* = 0.0042; Log-rank test). **(C - G)** Post-mortem brain histological and molecular analyses. **(C and D)** H&E and anti-WNT6 IHC staining were used as controls for GBM formation and successful long-term WNT6 silencing, respectively. **(E** - **G)** IHC for cell proliferation/viability markers Ki-67 and Cyclin D1 **(E)**, stem cell markers SOX2 and NESTIN **(F),** and anti-apoptotic marker BCL2 **(G).** Scale bars' values are shown in each microphotograph (20 µm or 50 µm).

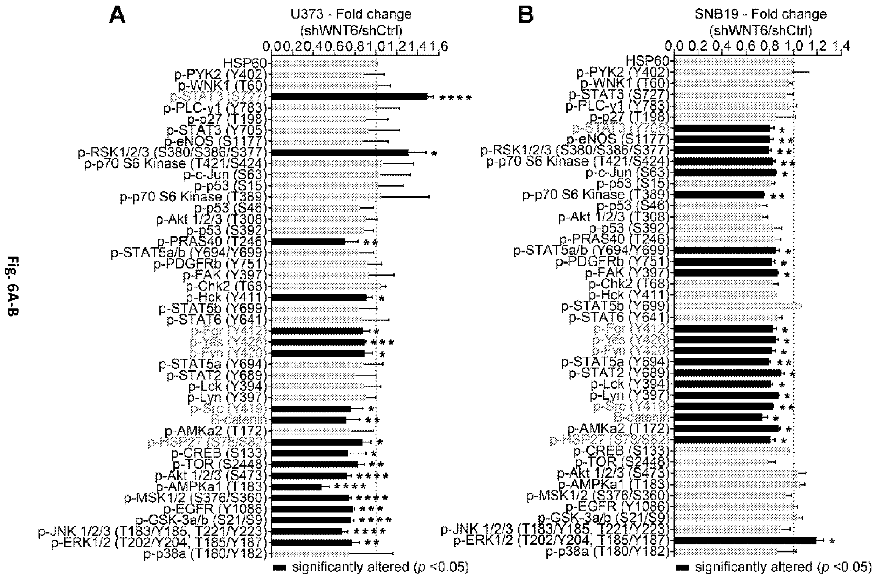

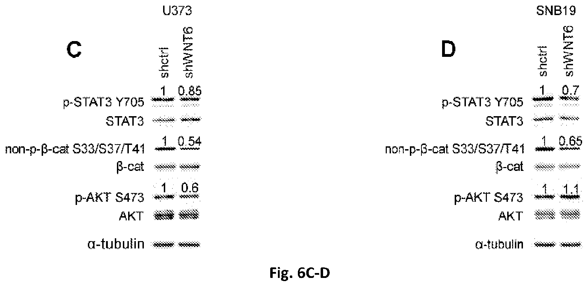

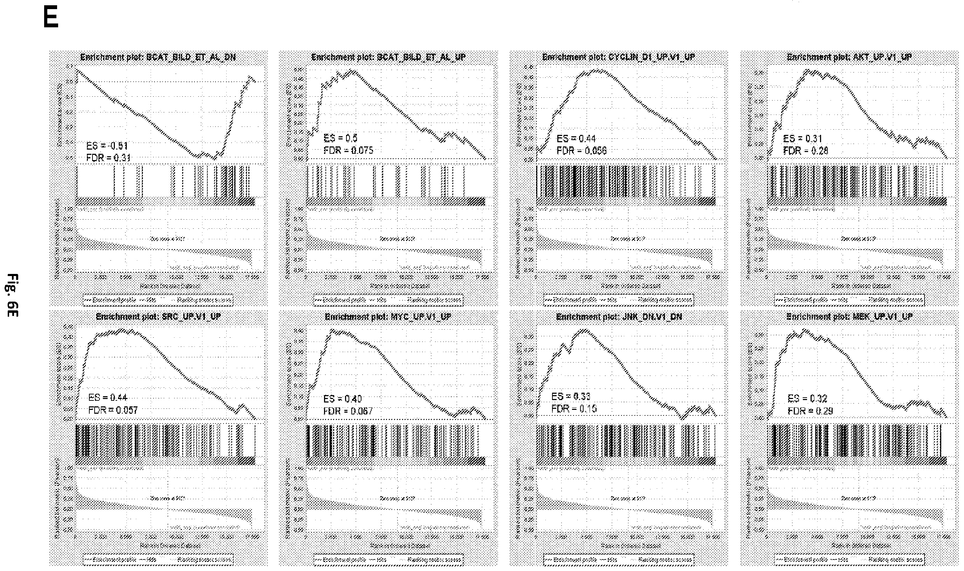

**Figure 6****.** WNT6 regulates the activation status of WNT, RTKs, and STAT pathways in GBM. Phospho-kinase arrays were performed to evaluate the phosphorylation status of cancer-related kinases in U373 **(A)** and SNB19 **(B).** Each array includes 2 technical replicates; 2 and 1 biological replicates were used for U373 and SNB19, respectively. Fold changes between phosphorylation levels of proteins in shWNT6 vs shCtrl cells are shown (means ± SDs). HSP60 was used as reference protein. Similar alterations in the phosphorylation of particular proteins for U373 and SNB19 cells are highlighted in red. *, *p* < 0.05; **, *p* < 0.01; ***, *p* < 0.005; ****, *p* < 0.0001; two-sided unpaired *t*-test. **(C and D)** The phosphorylation status of STAT3, β-catenin, and AKT, and their respective total forms were assessed in U373 **(C)** and SNB19 **(D)** shCtrl and shWNT6 cells by WB (images are representative of 3 independent assays; α-tubulin was used as reference protein for normalization - relative expression levels are shown above each band). **(E)** GSEA analyses identifying signatures of genes significantly associated (ES > 0.3, and FDR < 0.3) with *WNT6* in GBM patients from TCGA.

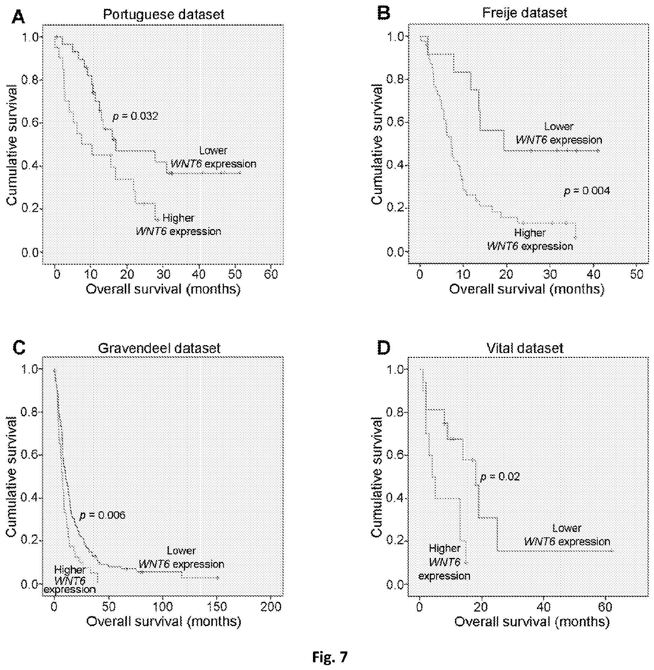

**Figure 7****.** *WNT6* expression is prognostically valuable in GBM patients. Kaplan-Meier survival curves of WNT6-low and WNT6-high GBM patients derived from qRT-PCR data **(A)** or microarray **(B-E)** data. **(A)** Portuguese dataset, n = 51, median OS 16.9 *vs* 10.4 months (low *vs* high *WNT6* expression, respectively; *p* = 0.032, Log-rank test). **(B)** Freije dataset, n = 59, median OS 9.6 *vs* 5 months (*p* = 0.006, Log-rank test). **(C)** Gravendeel dataset, n = 159, median OS 10.1 *vs* 6 months (*p* = 0.029, Log-rank test). **(D)** Vital dataset, n = 26, median OS 18 *vs* 4 months (*p* = 0.02, Log-rank test). **(E)** Proposed model for the functional, molecular and clinical impact of WNT6 in GBM. WNT6-high GBM cells present increased proliferation, invasion, migration, TMZ resistance and stem cell self-renewal capacities, which are accompanied by the activation of the WNT, SFK, RTK, STAT and AKT pathways. Clinically, WNT6-high tumors cause shorter overall survival (both in mouse GBM models and human patients).

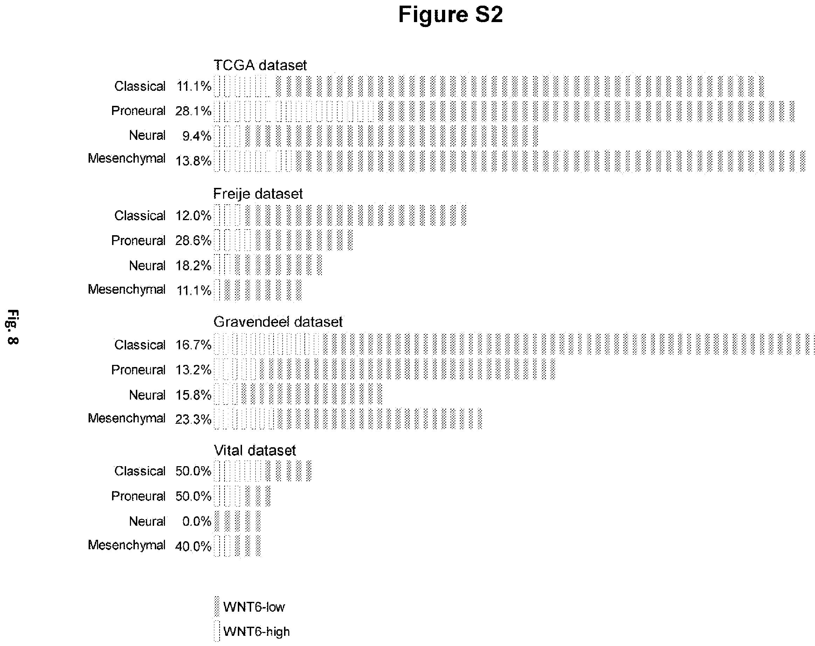

**Figure 8****.** *WNT6* expression is present in all 4 molecular subtypes of GBM. Distribution of WNT6-high patients among GBM molecular subtypes from the TCGA (n = 201), Freije (n = 59), Gravendeel (n = 159) and Vital (n = 26) datasets.

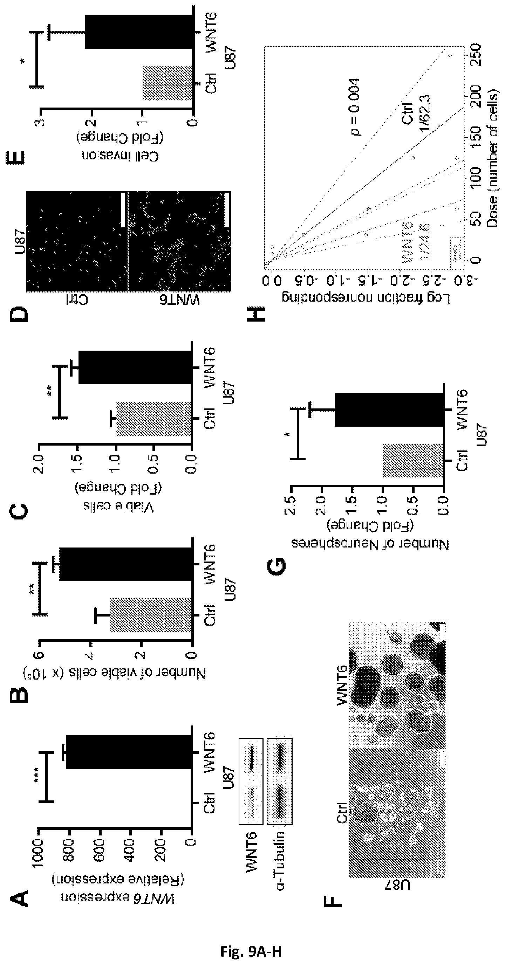

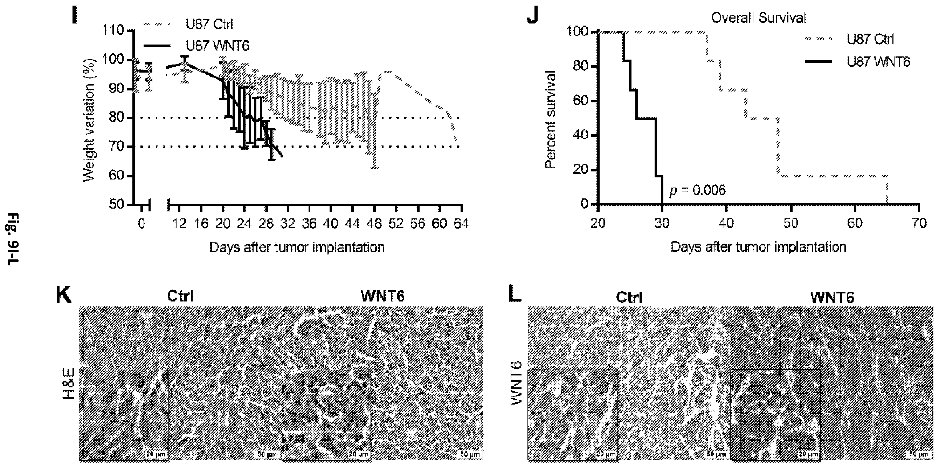

**Figure 9****.** WNT6 overexpression promotes GBM aggressiveness in vitro and in vivo. (A) The efficiency of WNT6 overexpression in U87 glioblastoma cells was analyzed by qRT-PCR (top) and WB (bottom). (Top) WNT6 expression levels were normalized to TBP. (Bottom); α-tubulin was used as reference protein. (B and C) Cell viability was measured by trypan blue (B) and MTS (C) assays in Ctrl and WNT6 cells. (D and E) Matrigel invasion assays were used to assess the cells' invasion capacity. Representative images (D; cell nuclei stained with DAPI; scale bar = 300 µm) and quantification (E) of invasive U87 Ctrl and WNT6 cells. (F) Representative images of U87 Ctrl/WNT6 neurospheres at day 10 after plating (40x magnification; scale bar = 200 µm). (G) Quantification of neurospheres formation. (H) Limiting dilution assays in U87-Ctrl (black) and U87-WNT6 (red) cells to assess their sphere-forming capacity. The trend lines represent the estimated active cell frequency (n = 3 independent assays; p = 0.004, likelihood ratio test). (I-L) U87 transfected cells were orthotopically injected in the brain of NSG mice (n = 6 per group). (I) Mice weight curves after tumor implantation. (J) Kaplan-Meier overall survival curves of mice (p = 0.006; Log-rank test). (K and L) Post-mortem brain histological and molecular analyses. H&E (K) and anti-WNT6 IHC staining (L) were used as controls for GBM formation and successful long-term WNT6 overexpression, respectively. Results represent data from at least 3 independent experiments (mean ± SD). *, p < 0.05; **, p < 0.01 and ***, p < 0.005 (otherwise stated, a two-sided unpaired t-test with Welch's correction being applied when homoscedasticity was not verified).

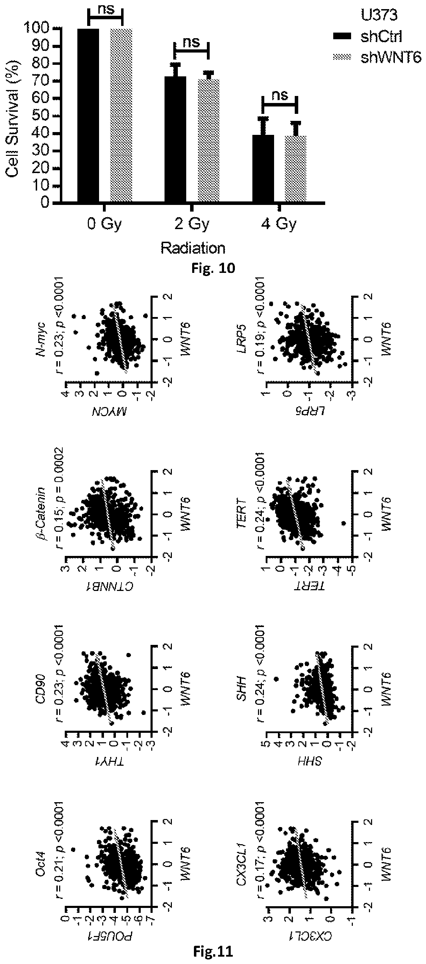

**Figure 10****.** WNT6 does not affect sensitivity of GBM cells to radiotherapy. U373 shCtrl and shWNT6 cells were treated with increasing doses of radiation (0, 2, and 4 Gy) and cell survival was analyzed by colony formation assays. ns = non-statistically significant (two-way ANOVA post-hoc Sidak's test).
**Figure 11****.** *WNT6* correlates with stem cell genes. Correlation graphs between *WNT6* expression (x-axis) and the expression of stem cell genes selected based on the heatmap in Figure 4 (y-axis; Pearson's correlation test r and p values are indicated).

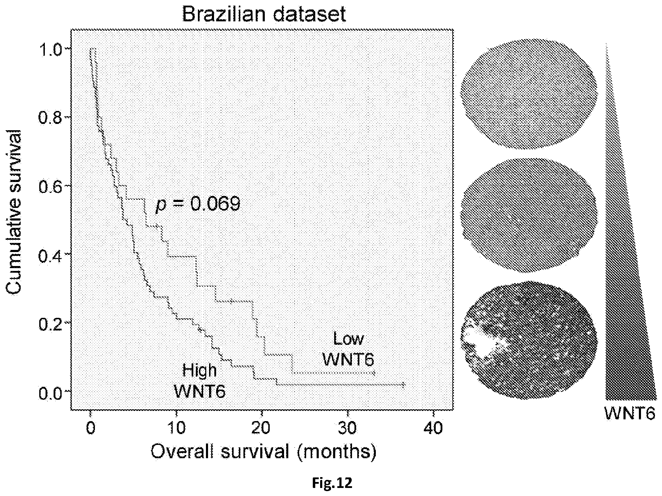

**Figure 12****.** Expression levels of WNT6 protein are prognostically valuable in GBM patients. Kaplan-Meier survival curves of WNT6-low and WNT6-high GBM patients derived from IHC (protein) data from a Brazilian dataset (n = 87; median OS 6.5 vs 3.8 months, low vs high WNT6 expression, respectively; p = 0.069, Log-rank test). Representative IHC microphotographs showing different levels of WNT6 protein expression in particular tumors are shown.

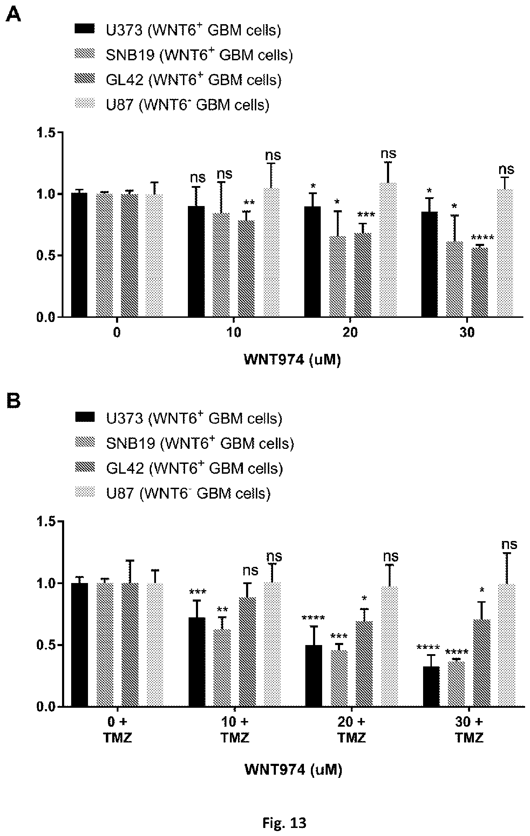

Figure 13. WNT6-positive GBM cell lines are more sensitive to WNT974 (an inhibitor of the WNT pathway) than a WNT6-negative GBM cell line. A. GBM cells positive to WNT6 (U373, SNB19, and GL42) and negative to WNT6 (U87) were exposed to increasing doses of WNT974 (0, 10, 20, or 30 µM). The viability of U87 cells (WNT6-negative) was not affected by WNT inhibition, in contrast to WNT6-positive GBM cells (U373, SNB19, and GL42) that are highly-sensitive. B. Cells were exposed to increasing doses of WNT974 (0, 10, 20, or 30 µM) and to a fixed dose of TMZ (temozolomide, the gold standard chemotherapeutic drug used for GBM patients; U373: 400 µM; SNB19: 800 µM; GL42: 200 µM; U87: 800 µM). While WNT6-positive cells presented a higher sensitivity to the combinatorial therapy, no effect was observed in the viability of U87 cells (negative for WNT6).

### Detailed Description

The present disclosure relates to a novel biomarker for use in human glioblastoma, namely to a compound inhibiting WNT6 biomarker for the treatment of glioblastoma with WNT6 over-expression, preferably proneural glioblastoma with WNT6 over-expression, classic glioblastoma with WNT6 over-expression, neural glioblastoma with WNT6 over-expression, or mesenchymal glioblastoma with WNT6 over-expression.

The present disclosure also relates to a WNT pathway inhibitor selected from the following list: vantictumab, GNF-1331, ipafricept, XAV-939, IWR-1, pyrvinium, DKN-01, IWP-2, WNT974, niclosamide, sulindac, NSC668036, J01-017a, PRI-724, ICG-001, Calphostin C, Toxoflavin, Xanthothricin, NCB-0846, Cercosporin, StAX-35, NVP-TNKS656, JW74, Foxy-5, OTSA 101, CWP232291, ETC 159, Rosmantuzumab, R04929097, MK0752, PF-03084014, or combinations thereof for use in a method of treating glioblastoma with WNT6 over-expression, pharmaceutical compositions and a kit for use in the treatment of glioblastoma.

In an embodiment, while high WNT6 expression levels were observed in different human cancer cell lines (1, 5), little is known about its specific role in tumors, particularly in GBM. In order to address this, first the gene expression array data from normal brains, lower-grade gliomas (LGG, WHO grades II and III) and GBM (WHO grade IV) patients deposited in TCGA were analysed. When compared to non-tumor samples, WNT6 was not overexpressed in any of the LGG patients (0/27), while 15.6% of GBM patients (89/572) presented high WNT6 levels (Figure 1A; p = 0.026). Concordantly, testing the protein levels of WNT6 by immunohistochemistry (IHC) in another dataset of glioma tissues (from Hospital Santo António; HSA) showed that only GBMs present high expression of WNT6 protein (16.3%; Figure 1B bottom; p = 0.037). WNT6 immunoreactivity expression in glioma showed to be mainly cytoplasmic (closed arrowheads), with a diffuse pattern where almost all tumor cells are positive (Figure 1B, b and c), or a more scattered pattern (Figure 1B, d). Tumor-infiltrating lymphocytes were negative for WNT6 expression (arrow; Figure 1B, e), with endothelial cells being negative or showing some faint immunoreactivity (open arrowheads; Figure 1B, b, c, d, and f). Together, these results show WNT6 mRNA and protein levels associate with high glioma grade, suggesting it may be important in the pathophysiology of glioma due to the overexpression of WNT6 in primary GBM tissues as follows.

The levels of *WNT6* expression were analysed among the GBM subtypes described by Verhaak *et al* (classical, mesenchymal, neural and proneural) in a total of 4 independent cohorts, totalizing 201 patients from TCGA, 59 from Freije, 159 from Gravendeel and 26 from Vital datasets. High levels of WNT6 were detected with no significant differences in subsets of patients of each GBM molecular subtype in all datasets (**Figure 1A** and **Figure 8**), suggesting WNT6 activation in GBM is independent of these molecular signatures. Thus, WNT6 high expression may occur in all molecular subtypes of GBM.

In an embodiment, to address whether WNT6 expression influences typical cancer hallmark features of GBM cells, WNT6 expression was silenced in U373MG and SNB19 cell lines by shRNA (**Figure 2A** and **B**), as well as overexpressed in U87MG cells, which do not express WNT6 endogenously (**Figure 9**). WNT6-low cells presented a significantly lower viability when compared with the corresponding WNT6-high counterparts (**Figure 2C** and D, and 9B and C). Similarly, WNT6 silencing significantly decreased the proliferation of GBM cells (**Figure 2E**). Considering GBM cells display remarkable migration and invasion potentials, it was tested how WNT6 affects these hallmark features of GBM. Wound healing assays and matrigel invasion assays showed that WNT6 silencing significantly decreases the capacity of GBM cells to migrate and invade (Figure 2F-K and **Figure 9D and E**). Together, these data suggest WNT6 is an important factor regulating several cancer hallmarks (e.g., viability, proliferation, invasion and migration), which might influence GBM aggressiveness. Furthermore, WNT6 has oncogenic functions and promotes GBM aggressiveness *in vitro.*

It was also tested whether WNT6 expression may have an impact in the resistance of GBM cells to clinically-relevant therapies, including temozolomide (TMZ) chemotherapy and radiation. Interestingly, both U373 and SNB19 WNT6-silenced GBM cells were significantly more sensitive to TMZ mediated cell death than control cells (**Figure 3A** and **B**). In contrast, WNT6 did not affect sensitivity of GBM cells to radiation treatment (**Figure 10**). Overall, these results suggest WNT6 promotes resistance of GBM cells to TMZ, with potential clinical implications as TMZ is still the standard-of-care for these patients. Thus, the WNT6 increases resistance to temozolomide chemotherapy in GBM cells.

In an embodiment, due to the critical roles of GBM stem cells (GSC) in the pathophysiology and clinical outcome of GBM patients, it was investigated how WNT6 may affect GBM cells' stemness. Interestingly, WNT6-silenced U373 and SNB19 cells showed prominent decreased levels of NESTIN and SOX2 proteins, two common markers of GSCs (**Figure 4A**). In addition, WNT6 expression resulted in significantly higher capacities of U373, SNB19 and U87 cells to form neurospheres when cultured under GSC conditions (**Figure 4B and C**, and **Figure 9Fand G**), functionally suggesting WNT6 contributes to the stem cell phenotype of GBM cells. Concordantly, WNT6 expression resulted in significantly increased neurosphere-forming frequency in a limiting dilution sphere-forming assay (1/3.81 for shCtrl vs 1/16.11 for shWNT6; *p* = 0.01, likelihood ratio test, Figure 4D for U373 cells; SNB19 cells with WNT6-silencing did not form spheres in the tested conditions - data not shown; 1/62.3 for U87-Ctrl vs 1/24.6 for U87-WNT6; p = 0.004, likelihood ratio test, **Figure 9H**), an assay that allows the quantification of cells with stem-like properties that are capable of forming a new neurosphere derived from a single cell.

In an embodiment, in addition to sphere-based stem cell assays, it was also tested U373 and SNB19 cells cultured under specific adherent conditions that allow GSC expansion (neurosphere medium supplemented with B27, EGF and FGF in laminin-coated wells). In these conditions, GBM cells acquired a more elongated phenotype (**Figure 4E**)**,** as previously described. Concordantly with our findings in "non-stem cell" conditions (**Figure 2C** and **D**), WNT6 silencing significantly impaired cell viability of U373 and SNB19 GBM cells grown in adherent stem cell-like conditions (**Figure 4F**). Globally, these data suggest WNT6 has an important functional role in the formation and maintenance of GSCs by promoting glioblastoma stem cell features.

To further translate the above-mentioned results to the clinical setting, it was evaluated correlations between the expression levels of *WNT6* and typical stem cell-related genes in GBM patient data from TCGA. Interestingly, heatmap analyses revealed common patterns of gene expression between some stem cell-related genes and *WNT6* **(****Figure 4G****),** and individual correlation graphs showed highly statistically significant correlations between *WNT6* and these genes (p < 0.0001; **Figure 4H****).** These data from GBM patients fit well with the *in vitro* findings, further suggesting WNT6 may be a critical stemness molecule in GBM. Thus, *WNT6* is associated with stem cell-related genes in GBM clinical samples. In an embodiment, the findings now disclosed were expanded to an *in vivo* model, U373 shCtrl/shWNT6 and U87 Ctrl/WNT6 cells were orthotopically-injected in the brains of NSG mice. Importantly, all mice bearing WNT6-low tumors presented significantly delayed evidences of GBM-related neurological symptoms and slower body weight loss than mice bearing WNT6-high tumors (**Figure 5A** **and** **Figure 9I**). This was accompanied by a significantly longer overall survival of U373-shWNT6 and U87-Ctrl mice as compared to mice injected with U373-shCtrl and U87-WNT6 cells, respectively (**Figure 5B** **and** **Figure 9J**)**,** implicating high levels of WNT6 as a negative prognostic biomarker in orthotopic GBM *in vivo* models. Post-mortem hematoxylin & eosin (H&E) analyses confirmed GBM formation in all animals (**Figure 5C** **and** **Figure 9K**), with a very invasive phenotype typical of GBM, and IHC confirmed long-term WNT6 silencing and overexpression in U373-shWNT6 and U87-WNT6 tumors, respectively (**Figure 5D** **and** **Figure 9L**). Histologically, shWNT6 GBM tumors also presented: i) lower proliferative activity, as observed by decreased Ki-67 and Cyclin D1 immuno-stainings (**Figure 5E**); ii) diminished expression of the stem cell marker SOX2, despite no alteration in the expression of NESTIN (**Figure 5F**); and iii) decreased expression of BCL2 protein, a critical anti-apoptotic molecule (**Figure 5G**)**.** Together, these data support WNT6 as a critical mediator of GBM aggressiveness *in vivo,* identifying some molecular mediators that might sustain this aggressive phenotype, in line with what was observed in the *in vitro* assays. Thus, high WNT6 levels accelerate GBM-associated death in intracranial mice models.

In an embodiment, to pinpoint the underlying molecular mechanisms by which WNT6 influences GBM aggressiveness, the activation status of a large variety of cancer-related kinases was evaluated in U373 and SNB19 cell lines using a human phospho-kinase antibody array (**Figure 6A** and **B**)**.** The phosphorylation levels of 4 proteins belonging to the non-receptor tyrosine Src family kinases [SFK; namely p-Fgr (Y412), p-Yes (Y426), p-Fyn (Y420) and p-Src (Y419)], and p-HSP27 (S78/S82), a member of the heat shock protein (HSP) family, were significantly decreased upon WNT6 silencing in U373 and SNB19 GBM cells (**Figure 6A** and **B**). In addition, the total levels of β-catenin were also decreased upon WNT6 silencing in both cell lines (**Figure 6A** and **B**).

Of note, other signaling pathways were concordantly affected upon WNT6 silencing in both cell lines, but at the level of different proteins. For example, the PI3K/AKT/mTOR pathway activation was decreased in U373 cells by directly affecting the phosphorylation of activating residues of AKT and mTOR, two of the major mediators of this pathway. While these specific proteins were not significantly altered in SNB19 cells, other downstream targets of this pathway, such as p70 S6K and eNOS, showed decreased phosphorylation levels, globally resulting in similar decreased signaling of the PI3K pathway. It is also interesting to note that STAT3 phosphorylation levels were altered in WNT6-silenced U373 and SNB19 cells, but at different residues: serine 727 (S727) phosphorylation was increased in U373 cells, while tyrosine 705 (Y705) phosphorylation was decreased in SNB19 cells (**Figure 6A** and **B**). Despite the contrasting phosphorylation levels of these STAT3 residues, the biological meaning is the same, as S727 phosphorylation inactivates, while Y705 phosphorylation activates STAT3 activity.

In addition to these signalling pathways concordantly affected in both cell lines, cell-line specific effects were also observed, such as alterations in the phosphorylation of JNK, GSK-3a/b, and EGFR in U373 cells, and of STAT2, STAT5a, STAT5a/b, PDGFRb, and c-Jun in SNB19 cells. Again, despite these examples of cell-specific effects, the silencing of WNT6 consistently resulted in a decreased activation status of these cancer-related pathways in GBM.

To further validate the results obtained in the phospho-kinase arrays, the phosphorylation status of 3 proteins was assessed by western blot (WB) and normalized to α-tubulin (reference protein; **Figure 6C** and **D**), as performed in the arrays. Interestingly, WBs show a slight decrease of p-STAT3 Y705 in U373 cells, and a more prominent decrease in SNB19 cells, as evidenced in the phospho-arrays. Similarly, a significant decrease of non-p-β-cat S33/S37/T41 levels upon WNT6 silencing was observed in both cell lines, while only U373 presented decreased p-AKT S473 phosphorylation levels, further validating the phospho-arrays data.

Together, these results suggest that WNT6 acts not only through β-catenin dependent WNT signalling, but also through the activation of downstream targets of SFKs/STAT pathways, highlighting WNT6 as a critical molecule involved in a variety of crucial signaling pathways in GBM, which associate with the observed oncogenic functional effects *in vitro* and *in vivo,* and reveal putative novel targets for therapeutic interventions in WNT6-high GBMs. Thus, WNT6 acts through β-catenin-dependent WNT signaling and activates downstream targets of SFKs/STAT pathways.

In an embodiment, to expand the WNT6-associated molecular mechanisms detected in *vitro* phosphorylation data (**Figure 6A-D**) to clinical samples of GBM, gene set enrichment analysis (GSEA) were performed to identify transcriptomic signatures reminiscent of *WNT6-*associated genes in GBM patients (**Figure 6E**)**.** The gene expression profiles of the GBM patients from TCGA (n = 573) were gathered, and *WNT6* expression used as a continuous label (*i.e.,* all genes were ranked based on their correlation with *WNT6*)*.* WNT6-positively correlated genes revealed to be significantly enriched for genes up-regulated upon WNT, AKT, SRC, MYC, JNK and MAPK signaling activation (all enrichment scores, ES > 0.3, and false discovery rates, FDR < 0.3; **Figure 6E**)**.** Concordantly, WNT6-negatively correlated genes were enriched for genes downregulated upon WNT signaling activation (ES > 0.3 and FDR < 0.3; **Figure 6E** top left). Overall, these transcriptomic results derived from GBM samples fit well with our *in vitro* phosphorylation data, further supporting the critical links between WNT6 and key oncogenic signaling pathways associated with glioma aggressiveness. Thus, *WNT6*-correlated genes are enriched for WNT, AKT, SRC, MYC, JNK and MAPK signaling pathways in GBM patients.

Since the data demonstrated critical roles of WNT6 in GBM aggressiveness *in vitro* and *in vivo,* and its association with GBM's key signaling pathways, it was also investigated the clinical significance of *WNT6* expression in a variety of GBM patient datasets. In the Portuguese dataset (Hospital Braga and Hospital Santa Maria, n = 51), high levels of *WNT6* mRNA were significantly associated with shorter overall survival (OS) of GBM patients (p = 0.032; log-rank test; **Figure 7A****).** Importantly, this association between high *WNT6* mRNA levels and poor prognosis in GBM patients was further validated in 3 additional independent datasets: Freije (n = 59), Gravendeel (n = 159) and Vital (n = 26) datasets (p = 0.006, p = 0.029, p = 0.02, respectively; **Figure 7B-D**). These findings were then validated in 407 GBM patients with available survival data from TCGA (Table 1), in which a multivariable Cox model showed a statistically significant association between higher values of *WNT6* expression and shorter OS of GBM patients (p = 0.030), independently of other prognostic variables, including patient age (p < 0.0001), Karnofsky performance status (KPS; p = 0.001), gender (p = 0.037) and the treatment with chemo- or radiotherapy (p < 0.0001; Table 1). Moreover, high levels of WNT6 protein expression, evaluated by IHC in a GBM Brazilian cohort (n = 87), also showed an evident trend for shorter OS (p = 0.069; **Figure 12**)**,** highlighting that both mRNA and protein levels of WNT6 may be used as a clinically-valuable biomarker. Together, these results suggest that *WNT6* expression may be a new prognostic factor useful for the stratification of GBM patients. Thus, high levels of *WNT6* expression associate with shorter overall survival in GBM patients.

**Table 1 - High levels of WNT6 expression are significantly associated with shorter survival of GBM patients (n = 407; microarray data.**

| | **Overall survival** | |
|---|---|---|
| | ***p*-value** | **Exp(B)** |
| ***WNT6* expression** | 0.03 | 1.288 |
| **Age** | <0.0001 | 1.029 |
| **KPS** | 0.001 | 0.984 |
| **Gender^{A,B}** | 0.037 | 0.771 |
| **Chemo- or Radiotherapy^{A,C}** | <0.001 | 0.192 |
| ^{A}Gender and Chemo- or Radiotherapy was used as categorical variables; | | |
| ^{B}Female (n=154) vs Male (n=253); | | |
| ^{C}Treatment (n=383) vs No treatment (n=24). | | |

Together, these results suggest that *WNT6* expression may be a new prognostic factor useful for the stratification of GBM patients.

In physiological conditions, the WNT pathway controls cell fate, proliferation, migration, polarity and death. Deregulated WNT pathway has been reported in several cancers, including GBM, and has been implicated in promoting resistance to current therapies. This increased resistance has been attributed to stem-cell features sustained by WNT signaling, such as increased proliferation and migration, and decreased adhesion. Although the importance of the WNT pathway has been explored in several tumor types, little is known about WNT6 in cancer (1-4). Indeed, to the best of our knowledge, this is the first study focusing on the roles and mechanisms of WNT6 in human gliomas. Due to its critical function during embryogenesis, deregulated expression of WNT6 in adults may favor tumorigenesis.

This is the first comprehensive study dissecting the functional roles and underlying molecular mechanisms mediated by WNT6 activation in human GBM, establishing it as a clinically-relevant prognostic biomarker (**Figures 7** **and** **12** and **Table 1**)**.** Due to its critical functions during embryogenesis, deregulated expression of WNT6 in adults may favor tumorigenesis (1-4). It is showed that the expression of WNT6 in gliomas is grade-dependent, at the gene and protein levels (**Figure 1**), similarly to what was previously described for other WNT ligands, thus associating WNT6 with increased malignancy of gliomas. In addition, it is demonstrated prominent effects of WNT6 on GBM cell viability, proliferation, glioma stem cell capacity, invasion, migration and sensitivity to TMZ-based chemotherapy, comprehensively implicating WNT6 as an important oncogenic factor in glioma (**Figures 2****,** **3** **4** **and** **9**). This is in agreement with the known effects of other WNT ligands in GBM. According to the definition of CSC, it is showed that WNT6 expression not only correlates with the expression of cancer-stem cell associated genes, but also functionally impacts on the self-renewal capacity of GSC (**Figures 4** **and** **9F-H**)**.** Concordantly with the functional effects, molecular data also showed WNT6 associates with typical GSC markers, both in GBM cells and patient samples.

Considering the global effects of WNT6 in GSC phenotypes (**Figures 4** **and** **9F-H**)**,** cell motility capacity (**Figures 2** **and** **9D-E**), and resistance to chemotherapy (**Figure 3**), it will be interesting to evaluate its potential value as a biomarker for tumor recurrence, which is of paramount importance to the clinical management of GBM as these patients recur almost universally after treatment. Importantly, it was demonstrated that WNT6 is a novel biomarker of GBM prognosis, as patients with WNT6-high tumors from a variety of independent datasets presented a significantly shorter OS than WNT6-low tumors (**Figure 7****, Table 1** and **Figure 12**), a clinical finding that is in agreement with the *in vivo* data (**Figures 5** **and** **9I-J**). This might be linked to the WNT6 oncogenic activities, such as increased invasion/migration, hampering complete tumor resection, increased tumor relapse probability due to its roles in GSC maintenance (**Figures 4** **and** **9F-H**), and the response to TMZ treatment (**Figure 3**), which is still the gold-standard chemotherapeutic drug used for GBM patients. These findings fit well with previous reports describing that WNT6 increases the chemo-resistance of gastric (1) and bladder (2) cancer, and associates with the overall and disease-free survivals of esophageal squamous cell carcinoma patients (3). Together, these data implicate WNT6 as a novel prognostic biomarker and putative therapeutic target for GBM patients. The fact that WNT6 levels can be easily assessed at the mRNA or protein levels with routine molecular biology techniques may also facilitate the widespread implementation of this biomarker in the clinical settings.

Taking into consideration that (i) the first step for designing novel therapeutic strategies requires not only the identification of new molecular targets, but also the understanding of their functional mechanisms, and (ii) nothing was still known on WNT6-mediated signaling mechanisms in GBM or any other cancer type, our phospho-kinase arrays provide the first data on the activation status of several cancer-related pathways that may be regulated by WNT6 **(****Figure 6****).** The effects observed in β-catenin in U373 and SNB19 cells clearly suggest that WNT6 might act through the canonical WNT pathway in GBM. In addition, WNT6 silencing led to reduced phosphorylation/activation states of 4 non-receptor tyrosine Src kinases (Fgr, Yes, Fyn and Src), which have been described to contribute to the activation of downstream targets of RTKs, such as EGFR, influencing glioma aggressiveness. WNT6 silencing also resulted in decreased phosphorylation of HSP27, an anti-apoptotic protein, associated with increased cell proliferation and decreased Fas-induced apoptosis in prostate cancer, and shown to mediate the activation of p38/ERK (MAPK) pro-survival signalling in cervical cancer cells. Nevertheless, other cell-line specific alterations in WNT6-silenced cells, namely in p-AKT, p-TOR and p-PRAS40 in U373 cells, clearly demonstrate the association between WNT6 and the activation of the PI3K/AKT/mTOR pathway, which is known to have a major role in the oncogenic process of GBM cells. Regarding p-STAT3 alterations upon WNT6 silencing, it was reported that reduced p-STAT3 S727 or increased p-STAT3 Y705 lead to more aggressive glioma tumors *in vivo* and enhance glioma proliferation and invasion *in vitro,* which also fits well with a WNT6-mediated oncogenic role in GBM. Importantly, GSEA data derived from clinical GBM samples further supported the molecular links between WNT6 and these cancer-related pathways. In this context, our results together with the reports associating the WNT pathway with the MAPK, PI3K and STAT3 pathways strongly support a role of WNT6 in the regulation of these cancer-related pathways and suggest that WNT6 might act, at least partly, through the canonical WNT signalling.

In summary, this work provides significant contributions to the neuro-oncology field, profiting from the understanding of *WNT6*-mediated signalling pathways and from the portrayal of the functional roles and clinical value of WNT6 in GBM, potentially impacting on the prognostic stratification and aiding in rationale treatment decisions for GBM patients.

All gene expression data from TCGA samples hybridized by the University of North Carolina, Lineberger Comprehensive Cancer Center, using Agilent G4502A 244K, were downloaded from TCGA (*https:*//*portal.gdc.cancer.gov*/), including 572 GBMs, 27 LGGs, and 10 unmatched non-tumoral patient samples. To prevent duplicated entries from the same patient - when more than one portion per patient was available - the median expression value was used. Three probe sets hit *WNT6* gene (A_23_P119916, A_32_P159877 and A_24_P208513). The provided value was pre-processed and normalized according to "level 3" specifications of TCGA. *WNT6* overexpression in glioma samples was considered when higher than in non-tumoral samples ("level 3" value ≥ 0.41). Clinical data of each patient was provided by the biospecimen core resources and includes information about patients' age at diagnosis, gender, KPS and days to death and to last follow-up.

*WNT6* expression microarray data from Freije (n = 59), Gravendeel n = 159, and Vital (n = 26) datasets of GBM patients were obtained in the GlioVis data portal. The top 17% of tumors expressing highest levels of *WNT6* were classified as WNT6-high in Freije (n = 10/59) and Gravendeel (n = 27/159) datasets, similarly to the cutoff used for TCGA, and the top 39% for the Vital (n = 10/26) dataset due to the small number of patients. Clinical data included GBM subtype classification, OS and vital status of the patients.

Glioma tumor specimens were obtained from patients who performed a craniotomy for tumor removal or stereotaxic biopsy at 4 different hospitals: Hospital Braga (HB), Hospital Santa Maria (HSM, Centro Hospitalar Lisboa Norte; samples requested from Biobanco-iMM, Lisbon Academic Medical Center, Lisbon, Portugal) and Hospital Santo António (HSA, Centro Hospitalar Porto), Portugal, and Hospital de Câncer de Barretos, Brazil. Samples from HB and HSM were reserved for RNA-based studies, transported in dry ice to the lab and stored at -80 °C. Samples from HSA and Brazil were formalin-fixed and paraffin-embedded and used for IHC analysis. Samples from Brazil were organized in triplicates into tissue microarrays (TMAs). Tumors were classified according to WHO 2007 and only patients with glial tumor histological diagnosis were included in the study.

Tissues sections for immunohistochemistry were deparaffinized and rehydrated by xylene and ethanol series. Sodium citrate buffer (10 mM, 0.05% Tween 20, pH 6) was used for antigen retrieval. Endogenous peroxidase activity was blocked with 3% H₂O₂ in TBS 1x for 10 min. Immunohistochemical staining was performed using the LabVision kit (UltraVision Large Volume Detection System Anti-polyvalent, HRP) according to the manufacturer's instructions. WNT6 (#ab50030, abcam; 1:450), Ki-67 (#550609, BD Biosciences; 1:200), Cyclin D1 (#2978, Cell Signaling; 1:100), SOX2 (#AB5603, EMD Millipore; 1:500), Nestin (#MAB5326, EMD Millipore; 1:100) and BCL2 (#2870, Cell Signaling; 1:200) antibodies were used. Concerning Ki-67 staining, before antigen retrieval, tissues were permeabilized using TBS-Tween 0.5%, for 10 min. DAB substrate (DAKO) was used as chromogen, followed by counterstaining with hematoxylin. The TMA from Brazil patients was blind-scored by a pathologist based on WNT6 staining intensity: 1 = weak, 2 = moderate and 3 = strong. The average of the triplicate spots was calculated and used for the survival analysis. An average above 2 was considered as WNT6 high expression.

Human GBM cell lines U373MG and U87MG (kindly provided by Dr. Joseph Costello, University of California, San Francisco) and SNB19 (obtained from German Collection of Microorganisms and Cell Cultures) were cultured in DMEM (Biochrom) supplemented with 10% Fetal Bovine Serum (FBS; Biochrom). Cells were maintained in a humidified atmosphere at 37 °C and 5% (v/v) CO₂. These conditions were maintained throughout the studies, unless otherwise stated. Testing for mycoplasma contamination was performed every month.

In an embodiment, the WNT6-silencing by shRNA in GBM cells was carried out as follows. U373MG and SNB19 cell lines were plated at 70 000 cell/well (24-well plate) and 100 000 cells/well (12-well plate), respectively. Cells were transfected with *WNT6* gene specific shRNA expression vector inserted in a pRS plasmid (TR308360, clones TI333434 and TI333435 from Origene; named U373 shWNT6 or SNB19 shWNT6, respectively) or with a scrambled negative control non-effective shRNA in a pRS plasmid (TR30012, Origene; named U373 shCtrl or SNB19 shCtrl). Lipofectamine 3000 (Invitrogen) transfection reagent was used according to manufacturer's recommendations (ratio plasmid/lipofectamine, 1:2.5). Stable transfection was achieved by selecting transfected clones with puromycin (1 µg/mL).

In and embodiment, the WNT6 overexpression in GBM cells was carried out as follows. U87MG cells were plated at 200 000 cell/well (6-well plate). Cells were transfected with the pcDNA-Wnt6 vector (gift from Dr. Marian Waterman; Addgene plasmid #35913) or with the respective empty vector (pcDNA3.2 GW delCMV; gift from Dr. Edward Hsiao; Addgene plasmid #29496). Lipofectamine 3000 (Invitrogen) transfection reagent was used according to manufacturer's recommendations (4 µL of Lipofectamine 3000 per 1 µg of DNA). Stable transfection was achieved by selecting transfected clones with geneticin (800 µg/mL).

In an embodiment, the qRT-PCR was carried out as follows. Total RNA was extracted from GBM patients' tissues and cell lines using the TRIzol method (Invitrogen) and cDNA from 1 µg of the total RNA was then synthesized (RT-Phusion Kit, Thermo Scientific). The levels of *WNT6* and *TBP* (reference gene) were assessed by quantitative reverse transcription-PCR (qRT-PCR; KAPA SYBR^{®} FAST qPCR Kit, KAPABIOSYSTEMS or PowerUp^{™} SYBR^{™} Green Master Mix, ThermoFisher Scientific, for cell lines and GBM patient's samples, respectively) with the following sets of primers: *WNT6* Fwd 5'-GACGAGAAGTCGAGGCTCTTT-3' and Rev 5'-CGAAATGGAGGCAGCTTCT-3'; *TBP* Fwd 5'-GAGCTGTGATGTGAAGTTTCC-3' and Rev 5'-TCTGGGTTTGATCATTCTGTAG-3'. The annealing temperature was 60 °C for both. Levels were determined based on the 2-_{ΔΔCt} method.

In an embodiment, the immunofluorescence (IF) studies were carried out as follows. U373 (shCtrl/shWNT6) and SNB19 (shCtrl/shWNT6) cells were plated in coverslips in DMEM supplemented with 10% FBS. Cells were fixed with paraformaldehyde 4% and then incubated in 1% BSA in PBS - 0.1% Tween for 1 hour. Afterwards, cells were incubated overnight at 4 °C with the primary antibody against WNT6 (ab50030, abcam; 1:1000) or SOX2 (AB5603, EMD Millipore, 1:300). Alexa fluor^{®} 594 goat anti-rabbit IgG (H+L; A-11012, ThermoFisher Scientific) secondary antibody was used at a dilution of 1:1 000 for 1 hour, at room temperature (RT), in the dark. Additionally, SOX2-coverslips were incubated with anti- NESTIN primary antibody (MAB5326, EMD Millipore, 1:100), for 1 hour at RT, in the dark. Next, Alexa Fluor^{®} 488 goat anti-mouse IgG (H+L; A-11001, ThermoFisher Scientific) secondary antibody was used at a dilution of 1:1000 for 1 hour, at RT, in the dark. DAPI (VECTASHIELD^{®} Mounting Medium with DAPI, Vector Laboratories) was used to stain the cell nucleus (blue) at a concentration of 1.5 µg/mL.

In an embodiment, cell viability assays were carried out as follows. Cell viability was determined 6 days after plating by both trypan blue and MTT (U373 and SNB19 cells) or MTS (U87 cells) assays. For trypan blue assay, cells were plated, in triplicate, at an initial density of 20 000 cells/well in 6-well plates. For MTT/MTS assay, U373 and SNB19 transfected cells were plated in 12-well plates, in triplicate, at an initial density of 6000 (for U373 and U87 cells) or 10 000 cells/well (SNB19). For the MTT assay, cells were incubated with 0.5 mg of MTT (ThermoFisher Scientific) per mL of PBS 1x, during up to 2 hours in a humidified atmosphere at 37 °C and 5% (v/v) CO₂. Subsequently, the formed crystals were dissolved using an acidified isopropanol solution (0.04 M HCl in isopropanol), and the absorbance was read at 570 nm. For the MTS assay, cells were incubated with 10% of the MTS solution (CellTiter 96^{®} AQueous One Solution Cell Proliferation Assay; Promega) in DMEM supplemented with 10% FBS, during up to 1h in a humidified atmosphere at 37 °C and 5% CO2. Absorbance was recorded at 490 nm.

In an embodiment, cell proliferation was carried out as follows. Cell proliferation was evaluated based on the measurement of BrdU incorporation during DNA synthesis (Cell Proliferation ELISA, BrdU colorimetric assay, Roche), as recommended by the manufacturer. Briefly, cells were cultured in DMEM (Gibco^{®}) supplemented with 10% FBS (Biochrom) in 96-well plates (cell density: 2 000 cells/well) for 4 days. After that, BrdU was added to the cells and they were re-incubated for 4 hours.

In an embodiment, the cell migration was carried out as follows. Ibidi 2-well inserts (Ibidi) with a cell-free gap were used in 12-well plates to evaluate cell migration by wound healing assay. U373 and SNB19 transfected cells were plated in triplicate, at an initial density of 70 000 and 60 000 cells in each side of the insert, respectively, and left to adhere overnight. After that, inserts were removed (0 hours' timepoint) and wells were washed with PBS to remove dead cells and debris. The migration of the cells into the gap was imaged (2 images/well) over hours until the wound closure was achieved (images were captured at the same localizations over time). The gap size (pixels) was measured using an automated software (beWound - Cell Migration Tool, v1.7, BESURG, Portugal; *www.besurg.com*) and manually verified and corrected when deemed necessary. At least 15 lines equally-spaced across the image and perpendicular to the gap main axis were used for increased measurement representativeness.

In an embodiment, cell invasion studies were performed as follows. Cell invasion was assessed using the BD BioCoat^{™} Matrigel^{™} Invasion Chambers (Corning^{®}) as recommended by the manufacturer. Briefly, cells were seeded into the top (insert) at the initial density of 50 000 cells/well (U373 and SNB19) or 25 000 cells/well (U87; 24-well chambers) in DMEM (Gibco^{®}) supplemented with 1% FBS. EGF was added to the medium of the lower chamber (DMEM supplemented with 10% FBS) at the concentration of 20 ng/mL, as chemoattractant. Cells were incubated for 22 hours. Henceforth, cells that invaded through the membrane were stained using VECTASHIELD Mounting Medium with DAPI (Vector Laboratories) and a picture from the entire membrane was obtained using the Olympus Upright BX61 microscope. The total cell number was counted with the help of the ImageJ software (version 1.49).

In an embodiment, cell death studies were carried out as follows. Cell death was evaluated after 6 days of treatment with TMZ (400 µM and 800 µM, for U373 and SNB19 transfected cells, respectively). TMZ treatment was applied 24 hours after plating and was renewed 2 days after. Cells were stained with Annexin V-FITC (BD Bioscience) and propidium iodide (PI; TermoFisher Scientific), according to manufacturer's protocol, followed by flow cytometry analysis. A total of at least 10 000 events were acquired. Results were analyzed using the FlowJo software (version 10).

In an embodiment, for clonogenic cell survival assay, cells were seeded at a density of 1 000 to 2 000 per 25-cmz flask, in triplicates. Twenty-four hours after seeding, irradiation was performed at RT in single exposure doses, delivered by a Novalis TX^{®} linear accelerator, with a nominal energy of 6 MV at a dose rate of about 400 cGy/min. Doses of 2 and 4 Gy were calculated using a dedicated treatment planning system to a depth of water of 1.5 cm. The plating efficiency (PE) represents the percentage of seeded cells that grew into colonies. Colonies with more than 50 cells were counted by microscopic inspection and PE, as well as the radiation-surviving fraction (PE of experimental group/PE of control group), were determined. Survival data were obtained with data from 3 independent experiments.

In an embodiment, the cell viability assay (MTT) under stem cell conditions was carried out as follows. Culture vessels were coated with natural mouse laminin (Invitrogen) for 3 hours at 37 °C or overnight at 4 °C at 10 µg/mL, as previously described. U373 and SNB19 transfected cells were plated in 12-well laminin-coated plates, in triplicate, at an initial density of 6000 and 10 000 cells per well, respectively. Cells were cultured in DMEM-F12 (Gibco) supplemented with 1x B27 (Invitrogen), 20 ng/mL of EGF (Invitrogen), and 20 ng/mL of basic fibroblast growth factor (b-FGF, Invitrogen). After 4 days in culture, cells were incubated with MTT as detailed above (section *Cell viability assays*)*.*

In an embodiment, the neurosphere formation assay was carried out as follows. U373, SNB19 and U87 transfected cells were plated at the maximum density of 4000 cells/mL in low-attachment 24-well plates (Corning). Cells were cultured in DMEM-F12 (Gibco) supplemented with 1x B27, 20 ng/mL of EGF, and 20 ng/mL of b-FGF. Neurospheres were supplemented with fresh media every 4-5 days (250 µL/well). The number of neurospheres was counted after 10 days for U373 and U87 cell lines and 15 days for the SNB19 ones.

In an embodiment, the limiting dilution assay was carried out as follows. Cell numbers of U373 and U87 transfected cells were adjusted to a starting concentration of 40 000 cells/mL and 160 000 cells/mL, respectively, from which multiple serial dilutions were performed and plated in low-attachment 96-well plates (Corning). At the end, cell densities ranged from 1000 to 1 cells per well in a final volume of 100 µL for U373 cells and from 4 000 to 8 cells per well for U87 cells. Cells were cultured in DMEM-F12 (Gibco) supplemented with 1x B27, 20 ng/mL EGF, and 20 ng/mL b-FGF. Cultures were disturbed only for media supplementation every 5 days. After 15 days, the fraction of wells not containing neurospheres was determined for each condition and plotted against the initially plated cellular density. Stem cell frequencies and statistical significance were calculated using the Extreme Limiting Dilution Assay (ELDA) software available at *http:*//*bioinf.wehi.edu.au*/*software*/*elda*/*.*

In an embodiment, the stereotactic orthotopic xenografts was performed as follows. NSG (*NOD.Cg-Prkdcscid Il2rgtm1Wjl*/SzJ) mice aged 4 months were anesthetized with a mixture of ketamine (75 mg/kg) and medetomidine (1 mg/kg) supplemented with Butorphanol (5 mg/kg) for its analgesic properties and placed into the digital 3-axis stereotaxic apparatus (Stoelting). A total of 5×10⁵ U373- or 2×10⁵ U87-transfected cells (resuspended in 5 µL PBS 1x) were injected into the brain striatum (1.8 mm medial-lateral right, -0.4 mm anterior-posterior and -2.5 mm dorsal-ventral from the bregma) using a 10 µL Hamilton syringe with a point style 4 beveled 26s-gauge needle. A total of 8 male and 6 female mice per group were used for U373- and U87-transfected cells, respectively. U373 and U87 cells were used as no reports were found for SNB19 tumorigenic capacity when orthotopically injected in immunocompromised mice. Mice were maintained under standard laboratory conditions, which includes artificial 12 hours' light/dark cycle, controlled ambient temperature (21 ± 1 °C) and a relative humidity of 50 - 60%. The confirmation of specified pathogen free health status of sentinels' mice maintained within the same animal room was performed according to FELASA guidelines. Experimental mice were always manipulated in a flow hood chamber, except during the surgery. Mice' body weight was evaluated 3 times per week, while general behavior and symptomatology were registered daily. The pre-established humane endpoint was performed when mice presented severe weight loss (> 30% from maximum weight). Death was used for Kaplan-Meyer representation.

In an embodiment, the human phospho-kinase antibody arrays (ARY003B; R&D systems) were used to simultaneously detect the relative levels of phosphorylation of 43 kinase phosphorylation sites and 2 related total proteins. Capture and control antibodies have been spotted in duplicate on nitrocellulose membranes. The entire protocol was done following manufacturer's recommendations. In this case, 400 µg of total protein from all cell lines (U373 shCtrl/shWNT6 and SNB19 shCtrl/shWNT6) was used. The obtained chemiluminescence was measured using the ChemiDoc imaging system (Biorad). Adjusted volume per spot (i.e. average pixel intensity of the spot times its area, corrected for the background) was measured using the Quantity One image analysis software. Background was eliminated using the local option. The average of each duplicated spot was calculated, normalized to HSP60 (reference protein) and used to determine the relative change in phosphorylated kinase proteins between shCtrl and shWNT6 conditions. Two sets of arrays were performed for U373 shCtrl/shWNT6, while only 1 was used for SNB19 shCtrl/shWNT6.

In an embodiment, western blot analysis was carried out as follows. Cells were washed with PBS 1x and removed by scratch in a lysis buffer containing 50 mM Tris pH 8, 2 mM EDTA pH 7.4, 0.1% SDS, 1% NP-40 and 150 mM NaCl, inhibitors of proteases 1x (B14001, Biotool) and phosphatases 1x (B15001, Biotool). After a 15-min incubation on ice, whole cell lysate was centrifuged at 13 000 xg for 15 min at 4 °C. The total protein concentration was determined from the obtained supernatant using the Pierce_{TM} BCA protein assay kit (ThermoFisher Scientific), according to manufacturer's protocol. Protein extracts (denatured and reduced; 20 µg/lane) were separated in a 10% SDS polyacrylamide gel by electrophoresis and transferred to nitrocellulose membranes (Hybond-C Extra, GE Healthcare Life Sciences), using the Trans-blot turbo transferring system (Bio-rad), following manufacturer's instructions. A protein ladder (Page ruler prestained protein ladder; Fermentas) was used to determine the approximate protein size, to monitor the progress of the electrophoretic run and the success of the transfer. Before starting the immunodetection, membranes were blocked with BSA (5% in TBS 1x), for 1 hour at RT. The immunodetection was achieved using antibodies against p-STAT3 Y705 (#9145, Cell Signaling; 1:2 000), STAT3 (#9132, Cell Signaling; 1:1 000), non-p-β-catenin S33/S37/T41 (#4270, Cell Signaling; 1:5 000), β-catenin (#610153, BD Biosciences; 1:2 000), p-AKT S473 (#4051, Cell Signaling; 1:1 000), AKT (#2920, Cell Signaling; 1:2 000), p-ERK T202/Y204 (#4370, Cell Signaling; 1:2 000), ERK (#4695, Cell Signaling; 1:1000) and α-tubulin (#sc-23948, Santa Cruz Biotechnology, 1:1000). Blots were revealed with peroxidase-conjugated secondary anti-mouse and anti-rabbit IgG antibodies (#sc-2031 and #sc-2004, respectively, Santa Cruz Biotechnology, 1:1 000), followed by enhanced chemiluminescence (ECL) solution (SuperSignal West Femto Chemiluminescent Substrate;Thermo Scientific). The obtained chemiluminescence was measured using the ChemiDoc imaging system (Biorad). If necessary, membranes were stripped using the Restore^{™} PLUS Western Blot Stripping Buffer (Thermo Fisher Scientific), following manufacturer's recommendations. As for the phospho-kinase arrays, adjusted volume per band was measured using the Quantity One image analysis software. Phosphorylated protein values were normalized to α-tubulin (reference protein).

In an embodiment, the gene set enrichment analysis (GSEA) was performed as follows. The raw expression data profile (Agilent G4502A 244K) of all GBM patients from TCGA (n = 573) was extracted as detailed above. GSEA (*www.broad.mit.edu*/*gsea*/) software was then used. In this work, a continuous phenotype profile was employed to find gene sets that correlate with *WNT6* (gene neighbors). Accordingly, the Pearson's correlation was used to score and rank the genes. Gene sets from the Molecular Signature Database (MSigDb) C6 collection were used. Default options were used, unless otherwise stated. Significant enrichments were considered when false discovery rate (FDR) < 0.30.

In an embodiment, the statistical analyses were performed as follows. Homoscedasticity was verified with the Levene's test and differences between groups were assessed by a two-sided unpaired t-test, with the Welch's correction being applied accordingly. For the wound healing assay and radiation treatment sensitivity assay, a two-way ANOVA followed by the post-hoc Sidak's test for multiple comparison testing was used. For the TMZ treatment cell death assay, a two-way ANOVA followed by the post-hoc Tukey's test for multiple comparison testing was used. The correlation between the expressions of *WNT6* and stem-related genes (from the gene ontology gene-set GO_Positive_regulation of stem cell_proliferation) was calculated using the Pearson's correlation coefficient (r). The effects of WNT6 on mice OS was assessed by log-rank test. These analyses were performed with the GraphPad Prism 6.01 software (GraphPad software, Inc.).

The Chi-square test was used to assess the difference between the distributions of tumors with high and low *WNT6* expression stratified for LGGs and GBMs. To evaluate the prognostic value of *WNT6* expression, univariate or multivariate analysis of survival were performed using, respectively, the log-rank test or the Cox proportional hazard model, where the potential confounding effect of some variables is considered. These analyses were made with SPSS 22.0 software (SPSS, Inc.).

For all statistical tests, significance was considered when p < 0.05.

The term "comprising" whenever used in this document is intended to indicate the presence of stated features, integers, steps, components, but not to preclude the presence or addition of one or more other features, integers, steps, components or groups thereof.

The above described embodiments are combinable.

The following claims further set out particular embodiments of the disclosure.

### List of sequences:

**SEQ ID 1: WNT6 mRNA**
   >uc002vjc.2 (WNT6) length=1703 
TCCGCAGGAGACACAGGCGCTGGCTGCCCCGTCCGCTCTCCGCCTCCGCCGCGCCCTCCTCGCCCGGGA
TGGGCCCCCCGCCGCCGCCGGATCCCTCGCCTCCCGGCCGCCGCCGTTGCGCTCGCCGCGCTCGCACT
GAAGCCCGGGCCCTCGCGCCGCGGTTCGCCCGCAGCCTCGCCCCCTGCCCACCCGGGCGGCCGTA
GGGCGGTCACGATGCTGCCGCCCTTACCCTCCCGCCTCGGgctgctgctgctgctcctgtgcCCGGCGCACG
TCGGCGGACTGTGGTGGGCTGTGGGCAGCCCCTTGGTTATGGACCCTACCAGCATCTGCAGGAAGGCA
CGGCGGCTGGCCGGGCGGCAGGCCGAGTTGTGCCAGGCTGAGCCGGAAGTGGTGGCAGAGCTAGCT
CGGGGCGCCCGGCTCGGGGTGCGAGAGTGCCAGTTCCAGTTCCGCTTCCGCCGCTGGAATTGCTCCAG
CCACAGCAAGGCCTTTGGACGCATCCTGCAACAGGACATTCGGGAGACGGCCTTCGTGTTCGCCATCAC
TGCGGCCGGCGCCAGCCACGCCGTCACGCAGGCCTGTTCTATGGGCGAGCTGCTGCAGTGCGGCTGCC
AGGCGCCCCGCGGGCGGGCCCCTCCCCGGCCCTCCGGCCTGCCCGGCACCCCCGGACCCCCTGGCCCC
GCGGGCTCCCCGGAAGGCAGCGCCGCCTGGGAGTGGGGAGGCTGCGGCGACGACGTGGACTTCGGG
GACGAGAAGTCGAGGCTCTTTATGGACGCGCGGCACAAGCGGGGACGCGGAGACATCCGCGCGTTGG
TGCAACTGCACAACAACGAGGCGGGCAGGCTGGCCGTGCGGAGCCACACGCGCACCGAGTGCAAATG
CCACGGGCTGTCGGGATCATGCGCGCTGCGCACCTGCTGGCAGAAGCTGCCTCCATTTCGCGAGGTGG
 
GCGCGCGGCTGCTGGAGCGCTTCCACGGCGCCTCACGCGTCATGGGCACCAACGACGGCAAGGCCCT
GCTGCCCGCCGTCCGCACGCTCAAGCCGCCGGGCCGAGCGGACCTCCTCTACGCCGCCGATTCGCCCG
ACTTCTGCGCCCCCAACCGACGCACCGGCTCCCCGGCACGCGCGGTCGCGCCTGCAATAGCAGCGCC
CCGGACCTCAGCGGCTGCGACCTGCTGTGCTGCGGCCGCGGGCACCGCCAGGAGAGCGTGCAGCTCG
AAGAGAACTGCCTGTGCCGCTTCCACTGGTGCTGCGTAGTACAGTGCCACCGCTGCCGTGTGCGCAAG
GAGCTCAGCCTCTGCCTGTGACCCGCCGCCCGGCCGCTAGACTGACTTCGCGCAGCGGTGGCTCGCAC
CTGTGGGACCTCAGGGCACCGGCACCGGGCGCCTCTCGCCGCTCGAGCCCAGCCTCTCCCTGCCAAAG
CCCAACTCCCAGGGCTCTGGAAATGGTGAGGCGAGGGGCTTGAGAGGAACGCCCACCCACGAAGGCC
CAGGGCGCCAGACGGCCCCGAAAAGGCGCTCGGGGAGCGTTTAAAGGACACTGTACAGGCCCTCCCT
CCCCTTGGCCTCTAGGAGGAAACAGTTTTTTAGACTGGAAAAAAGCCAGTCTAAAGGCCTCTGGATACT
GGGCTCCCCAGAACTGCTGGCCACAGGATGGTGGGTGAGGTTAGTATCAATAAAGATATTTAAACCAC
CA
**SEQ ID 2: WNT6 protein**

MLPPLPSRLGLLLLLLLCPAHVGGLWWAVGSPLVMDPTSICRKARRLAGRQAELCQAEPEVVAELARGARL
GVRECQFQFRFRRWNCSSHSKAFGRILQQDIRETAFVFAITAAGASHAVTQACSMGELLQCGCQAPRGRA
PPRPSGLPGTPGPPGPAGSPEGSAAWEWGGCGDDVDFGDEKSRLFMDARHKRGRGDIRALVQLHNNEA
GRLAVRSHTRTECKCHGLSGSCALRTCWQKLPPFREVGARLLERFHGASRVMGTNDGKALLPAVRTLKPPG
RADLLYAADSPDFCAPNRRTGSPGTRGRACNSSAPDLSGCDLLCCGRGHRQESVQLEENCLCRFHWCCVV
QCHRCRVRKELSLCL

### References

1. Yuan G, Regel I, Lian F, Friedrich T, Hitkova I, Hofheinz RD, Strobel P, Langer R, Keller G, Rocken C, et al. WNT6 is a novel target gene of caveolin-1 promoting chemoresistance to epirubicin in human gastric cancer cells. Oncogene.2013;32(3):375-87*.*
2. Fan Y, Shen B, Tan M, Mu X, Qin Y, Zhang F, and Liu Y. Long non-coding RNA UCA1increases chemoresistance of bladder cancer cells by regulating Wnt signaling. TheFEBS journal. 2014;281(7):1750-8.
3. Zhang L, Yuan G, Fang Y, Qiu M, Lin J, Sun J, and Yang D. Increased WNT6 expression in tumor cells predicts unfavorable survival in esophageal squamous cell carcinoma patients. International journal of clinical and experimental pathology. 2015;8(9):11421-7.
4. Galbraith RL, Poole EM, Duggan D, Muehling J, Hsu L, Makar K, Xiao L, Potter JD, and Ulrich CM. Polymorphisms in WNT6 and WNT10A and colorectal adenoma risk.Nutrition and cancer. 2011;63(4):558-64.
5. Kirikoshi H, Sekihara H, and Katoh M. WNT10A and WNT6, clustered in human chromosome 2q35 region with head-to-tail manner, are strongly coexpressed in SW480 cells. Biochemical and biophysical research communications. 2001;283(4):798-805.

## Claims

1. A compound for use in the treatment of glioblastoma with WNT6 over-expression, wherein the compound is selected from the following list:
vantictumab, GNF-1331, ipafricept, XAV-939, IWR-1, pyrvinium, DKN-01, IWP-2, WNT974, niclosamide, sulindac, NSC668036, J01-017a, PRI-724, ICG-001, Calphostin C, Toxoflavin, Xanthothricin, NCB-0846, Cercosporin, StAX-35, NVP-TNKS656, JW74, Foxy-5, OTSA 101, CWP232291, ETC 159, Rosmantuzumab, RO4929097, MK0752, PF-03084014, or combinations thereof.

2. The compound according to the previous claim for use in the treatment of proneural glioblastoma with WNT6 over-expression, classic glioblastoma with WNT6 over-expression, neural glioblastoma with WNT6 over-expression, or mesenchymal glioblastoma with WNT6 over-expression.

3. The compound for use according to the previous claims 1-2 wherein said compound is selected from the following list vantictumab, GNF-1331, DKN-01, WNT974, NSC668036, J01-017a, PRI-724, Calphostin C, Toxoflavin, Xanthothricin, NCB-0846, Cercosporin, StAX-35, NVP-TNKS656, JW74, OTSA 101, CWP232291, ETC 159, Rosmantuzumab, , or combinations thereof.

4. The compound for use according to the previous claim wherein the compound is WNT974.

5. Pharmaceutical composition for use in the treatment of glioblastoma with WNT6 over-expression, comprising the compounds described in any of the previous claims and a pharmaceutical acceptable excipient.

6. Kit for use in a method of treating glioblastoma with WNT6 over-expression, comprising the pharmaceutical composition or compound according to any of the previous claims, preferably for the treatment of proneural glioblastoma with WNT6 over-expression, classic glioblastoma with WNT6 over-expression, neural glioblastoma with WNT6 over-expression, or mesenchymal glioblastoma with WNT6 over-expression.

## Patentansprüche

1. Eine Zusammensetzung zur Verwendung bei der Behandlung von Glioblastom mit WNT6-Überexpression, wobei die Zusammensetzung aus der folgenden Liste ausgewählt ist:
Vantictumab, GNF-1331, Ipafricept, XAV-939, IWR-1, Pyrvinium, DKN-01, IWP-2, WNT974, Niclosamid, Sulindac, NSC668036, J01-017a, PRI-724, ICG-001, Calphostin C, Toxoflavin, Xanthothricin, NCB-0846, Cercosporin, StAX-35, NVP-TNKS656, JW74, Foxy-5, OTSA 101, CWP232291, ETC 159, Rosmantuzumab, RO4929097, MKO752, PF-03084014, oder Kombinationen davon.

2. Die Zusammensetzung nach dem vorangehenden Anspruch zur Verwendung bei der Behandlung von proneuralen Glioblastomen mit WNT6-Überexpression, klassischen Glioblastomen mit WNT6-Überexpression, neuralen Glioblastomen mit WNT6-Überexpression oder mesenchymalen Glioblastomen mit WNT6-Überexpression.

3. Die Zusammensetzung zur Verwendung nach den vorangehenden Ansprüchen 1-2, wobei die genannte Zusammensetzung aus der folgenden Liste ausgewählt ist, Vantictumab, GNF-1331, DKN-01, WNT974, NSC668036, J01-017a, PRI-724, Calphostin C, Toxoflavin, Xanthothricin, NCB-0846, Cercosporin, StAX-35, NVP-TNKS656, JW74, OTSA 101, CWP232291, ETC 159, Rosmantuzumab, oder Kombinationen davon.

4. Die Zusammensetzung zur Verwendung nach dem vorangehenden Anspruch, wobei die Zusammensetzung WNT974 ist.

5. Pharmazeutische Verbindung zur Verwendung bei der Behandlung von Glioblastom mit WNT6-Überexpression, umfassend die in einem der vorangehenden Ansprüche beschriebenen Zusammensetzungen und einen pharmazeutisch verträglichen Hilfsstoff.

6. Kit zur Verwendung in einem Verfahren zur Behandlung von Glioblastom mit WNT6-Überexpression, umfassend die pharmazeutische Verbindung oder Zusammensetzung nach einem der vorhergehenden Ansprüche, bevorzugt zur Behandlung von proneuralen Glioblastomen mit WNT6-Überexpression, klassischen Glioblastomen mit WNT6-Überexpression, neuralen Glioblastomen mit WNT6-Überexpression oder mesenchymalen Glioblastomen mit WNT6-Überexpression.

## Revendications

1. Un composé destiné à être utilisé pour le traitement du glioblastome avec surexpression WNT6, dans lequel le composé est choisi parmi la liste suivante :
vantictumab, GNF-1331, ipafricept, XAV-939, IWR-1, pyrvinium, DKN-01, IWP-2, WNT974, niclosamide, sulindac, NSC668036, J01-017a, PRI-724, ICG-001, Calphostine C, Toxoflavine, Xanthothricine, NCB-0846, Cercosporine, StAX-35, NVP-TNKS656, JW74, Foxy-5, OTSA 101, CWP232291, ETC 159, Rosmantuzumab, RO4929097, MK0752, PF-03084014, ou des combinaisons de ceux-ci.

2. Le composé selon la revendication précédente destiné à être utilisé dans le traitement du glioblastome proneural avec surexpression WNT6, du glioblastome classique avec surexpression WNT6, du glioblastome neural avec surexpression WNT6, ou du glioblastome mésenchymateux avec surexpression WNT6.

3. Le composé destiné à être utilisé selon les revendications précédentes 1-2 dans lequel ledit composé est choisi parmi la liste suivante, vantictumab, GNF-1331, DKN-01, WNT974, NSC668036, J01-017a, PRI-724, Calphostine C, Toxoflavine, Xanthothricine, NCB-0846, Cercosporine, StAX-35, NVP-TNKS656, JW74, OTSA 101, CWP232291, ETC 159, Rosmantuzumab, ou des combinaisons de ceux-ci.

4. Le composé destiné à être utilisé selon la revendication précédente dans lequel le composé est le WNT974.

5. Composition pharmaceutique destiné à être utilisé dans le traitement du glioblastome avec surexpression WNT6, comprenant les composés décrits dans l'une quelconque des revendications précédentes et un excipient pharmaceutique acceptable.

6. Kit destiné à être utilisé dans un procédé de traitement de glioblastome avec surexpression WNT6, comprenant la composition pharmaceutique ou le composé selon l'une quelconque des revendications précédentes, préférablement pour le traitement du glioblastome proneural avec surexpression WNT6, du glioblastome classique avec surexpression WNT6, du glioblastome neural avec surexpression WNT6, ou du glioblastome mésenchymateux avec surexpression WNT6.
